# EUROPEAN PATENT APPLICATION

(11) **EP 4 431 514 A1**
(43) Date of publication of application: **18.09.2024**
(21) Application number: 22892730.7
(22) Date of filing: 07.11.2022
(51) Int. Cl.: C07D 517/04, C30B 19/00, H01B 1/12, H01B 5/14, H01B 13/00, C30B 29/54, G01N 23/20, C07D 495/04

(54) **MOLECULAR METAL-BASED SINGLE CRYSTALLINE THIN FILM, COMPONENT HAVING SAME, AND METHOD FOR MANUFACTURING SINGLE CRYSTALLINE THIN FILM**

(30) Priority: 10.11.2021 JP 2021182911; 28.12.2021 JP 2021213585; 16.09.2022 JP 2022147972
(71) Applicant: National Institute for Materials Science, Tsukuba-shi, Ibaraki 305-0047 (JP)
(72) Inventor: KOBAYASHI Yuka, Tsukuba-shi, Ibaraki 305-0047 (JP); HIRATA Kazuto, Tsukuba-shi, Ibaraki 305-0047 (JP); TAKAHASHI Megumi, Tsukuba-shi, Ibaraki 305-0047 (JP); NISHIO Naoko, Tsukuba-shi, Ibaraki 305-0047 (JP); TAKEDA Yoshihiko, Tsukuba-shi, Ibaraki 305-0047 (JP); SATO Rodrigo, Tsukuba-shi, Ibaraki 305-0047 (JP); SONG Rui, Tsukuba-shi, Ibaraki 305-0047 (JP)
(74) Representative: J A Kemp LLP
(86) International application number: PCT/JP2022/041372
(87) International publication number: WO 2023/085231

(57) **Abstract**

Provided is a molecular metal-based single crystalline thin film having high electrical conductivity and high light transmittance. The molecular metal-based single crystalline thin film has a light transmittance of 80% or more at a wavelength of 360-830 nm, wherein the molecular metal is a compound represented by Formula (I).

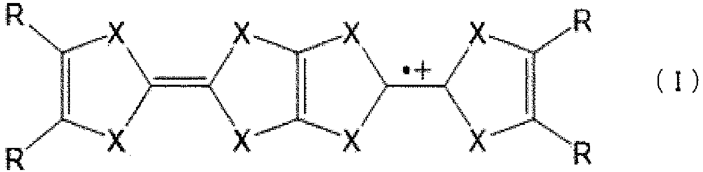

In Formula (I), X represents at least one atom selected from the group consisting of S, O, and Se, the plurality of X's may be the same or different, R represents a hydrogen atom or a monovalent substituent, the plurality of R's may be the same or different, provided that at least one among the R's is at least one group selected from the group consisting of groups represented by Formula (II), and the R's on adjacent atoms may be linked to each other to form a ring.

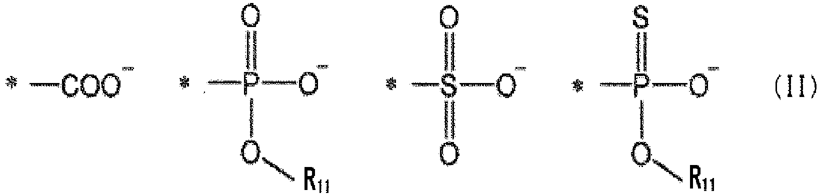

In Formula (II), * represents a bonding position, and R₁₁ represents a hydrogen atom or a C1-4 alkyl group.

## Description

### TECHNICAL FIELD

### (Cross-Reference to Related Applications)

This application claims priority to and the benefit of Japanese Patent Application No. 2021-182911 filed on November 10, 2021, Japanese Patent Application No. 2021-213585 filed on December 28, 2021, and Japanese Patent Application No. 2022-147972 filed on September 16, 2022 (hereinafter, they may be referred to as "P1", "P2", and "P3", respectively).

### (Technical Field)

The present invention relates to a single-component organic molecular metallic single crystalline thin film and a method for manufacturing the same. The present invention also relates to a single crystalline thin film of a synthetic metal, a component including the same, and a method for manufacturing a single crystalline thin film. In the present description, a single-component organic molecular metal, an organic molecular metal, TED, and/or a synthetic metal may be referred to as a molecular metal.

### BACKGROUND ART

Organic substances are basically insulating, but molecular metals that exhibit electrical conductivity (hereinafter also referred to as "conductivity") by use of a doping agent such as an oxidation or reduction reagent are known. Unfortunately, mixing of the doping agent may cause a decrease in stability of a compound, and the doping agent itself may adversely affect other parts of a product, which significantly reduces the applicability of organic electronic materials. Meanwhile, tetrathiafulvalene derivatives disclosed in, for example, Patent Literatures 1 to 4 and Non-Patent Literatures 1 and 2 are a molecular metal having electrical conductivity without the necessity of addition of a doping agent due to the superiority of their molecular structure.

### CITATION LIST

### PATENT LITERATURES

PATENT LITERATURE 1: JP 5943285 B
PATENT LITERATURE 2: JP 6145660 B
PATENT LITERATURE 3: JP 2020-15680 A
PATENT LITERATURE 4: JP 2020-07253 A

### NON-PATENT LITERATURES

NON-PATENT LITERATURE 1: Nature Mat. 2017, 16, 109
NON-PATENT LITERATURE 2: Chem. Sci. 2020, 11, 11699

### SUMMARY OF INVENTION

### TECHNICAL PROBLEMS

If a thin film having high electrical conductivity, high light transmissivity, and ultra-thin film properties can be developed using the synthetic metal (molecular metal) disclosed in any of Patent Literatures 1 to 4 and Non-Patent Literatures 1 and 2, application to various devices such as a transparent electrode becomes possible.

However, since the powdery (powdery microcrystalline) synthetic metals disclosed in Patent Literatures 1 to 4 and Non-Patent Literature 1 exhibit grain boundary resistance, the highest electrical conductivity of the substance has not been obtained. In addition, it is difficult to make a thin film of the powdery synthetic metal on a nanometer scale, and the obtained thin film had a film thickness on a micrometer scale. Moreover, since the thin film was not a single crystalline thin film but an amorphous thin film, it had an electrical conductivity that was several orders of magnitude lower than that of a normal conductor.

Meanwhile, a single crystal of a synthetic metal is reported in Non-Patent Literature 2. The single crystalline synthetic metal disclosed in Non-Patent Literature 2 exhibits, according to measurement by a four-terminal method, a top-class high electrical conductivity among organic conductors, that is, 2300 S/cm at room temperature. However, the single crystal disclosed in Non-Patent Literature 2 has a large absorption band in the visible light region and is colored in reddish brown, so that it has very low transparency.

The present invention aims to solve the above-mentioned problems, and provides a single crystalline thin film of a molecular metal having high electrical conductivity, high light transmissivity, and ultra-thin film properties.

### SOLUTIONS TO PROBLEMS

Solutions to the problems based on the contents of P1 or P3 are as follows.
[1] A single-component organic molecular metallic single crystalline thin film (the word refers to a single crystalline thin film of a molecular metal that is composed of a single component, and may be simply referred to as a "single crystalline thin film of a molecular metal" in the present description) of the present invention, which is based on the contents of P1 or P3, comprises any one compound represented by a general formula selected from the following: 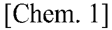 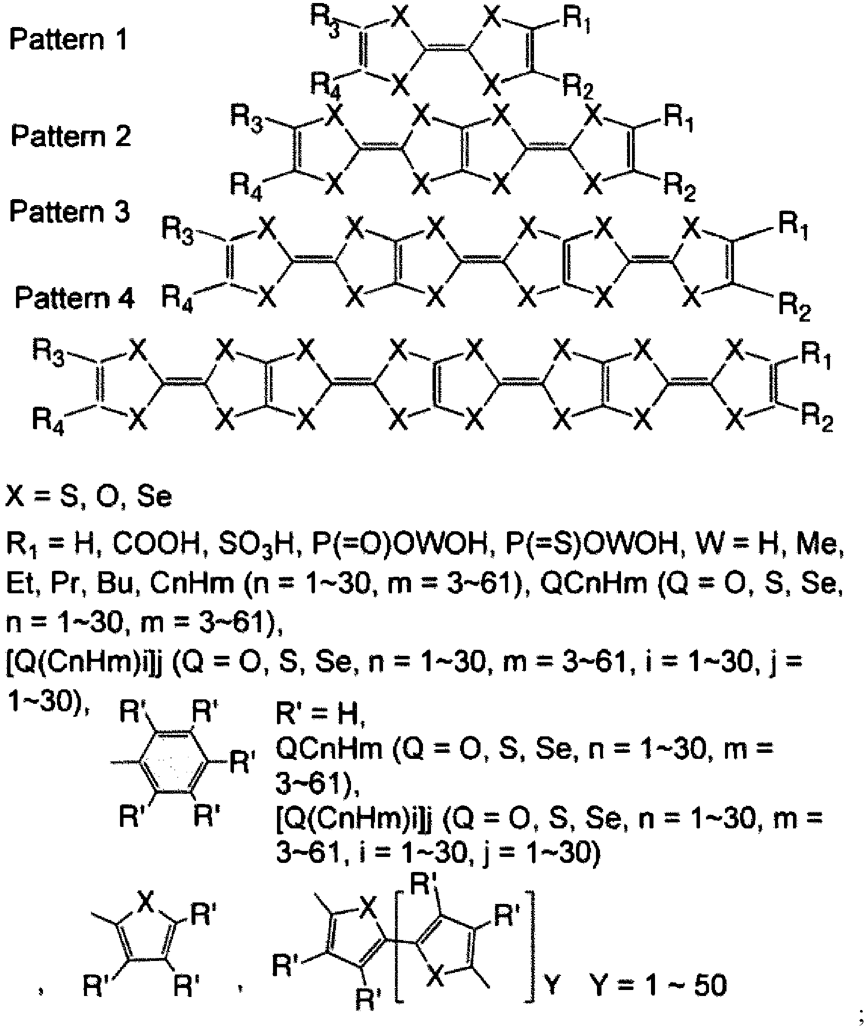 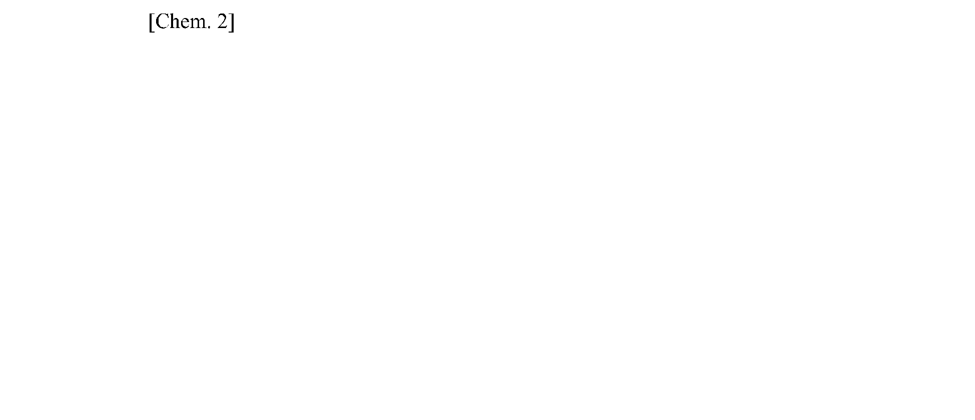 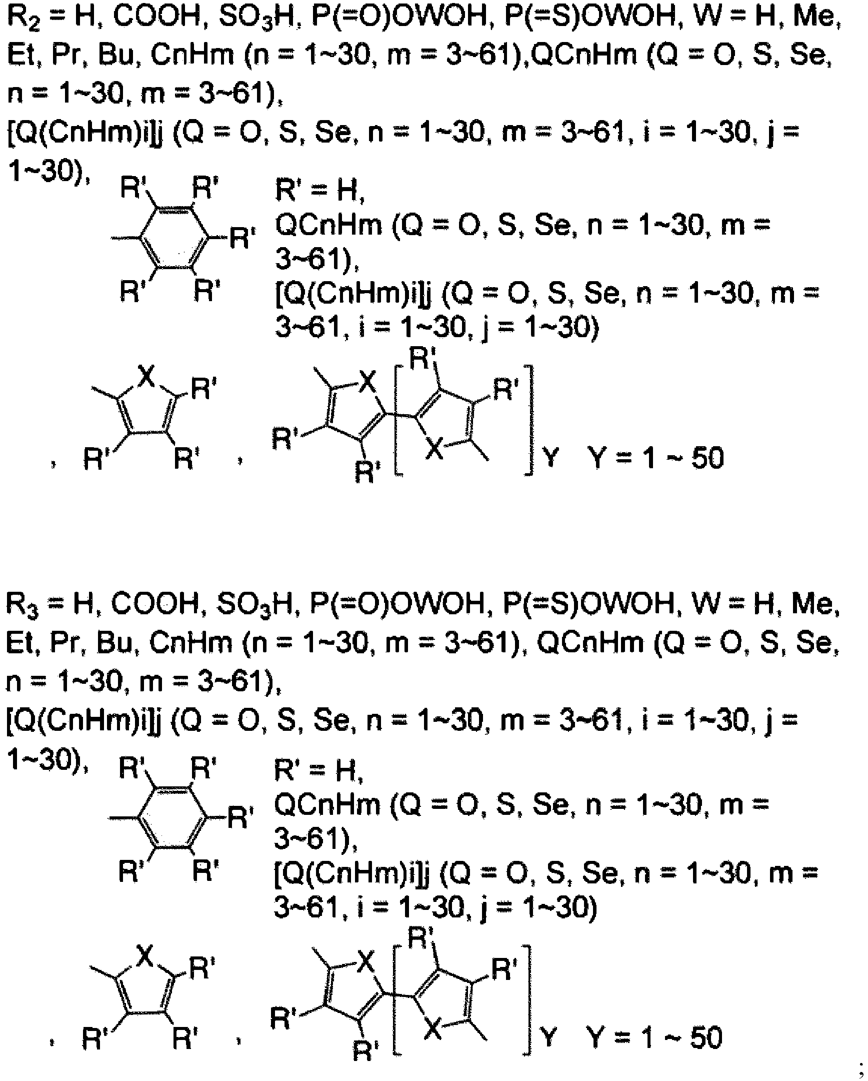 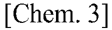 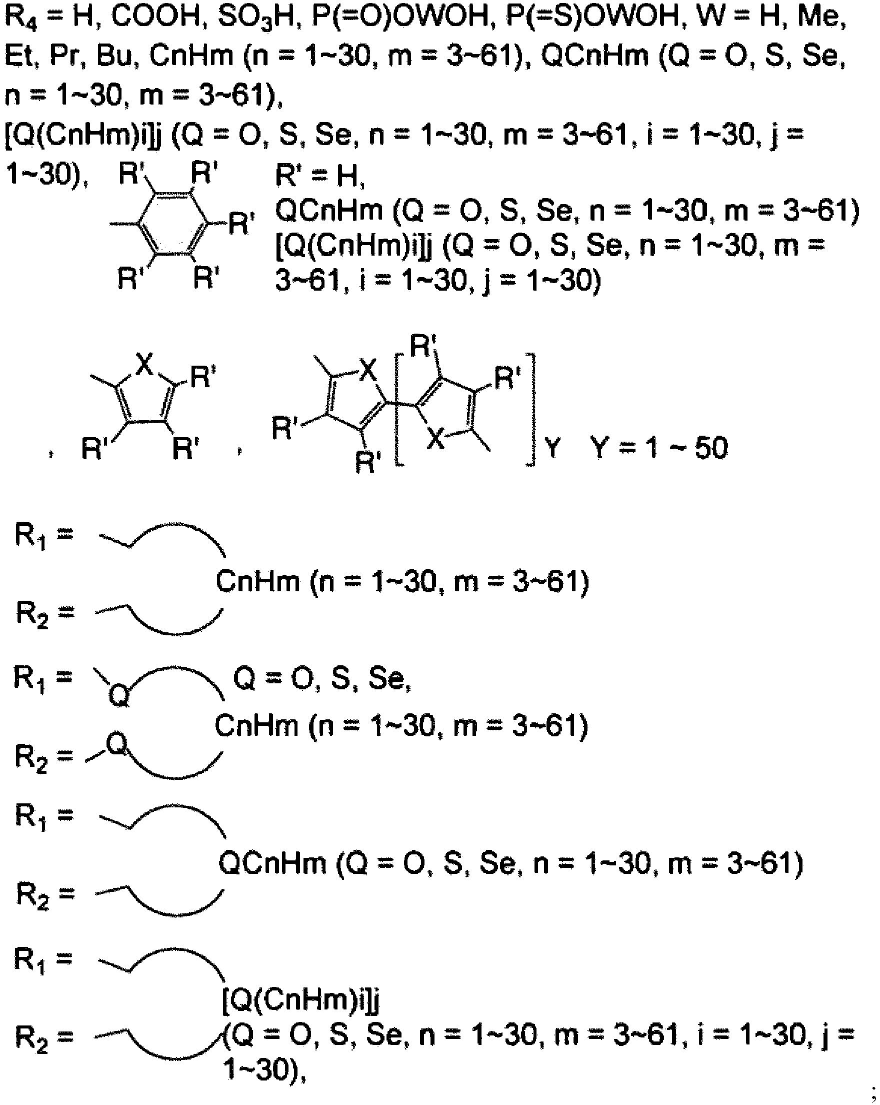 and 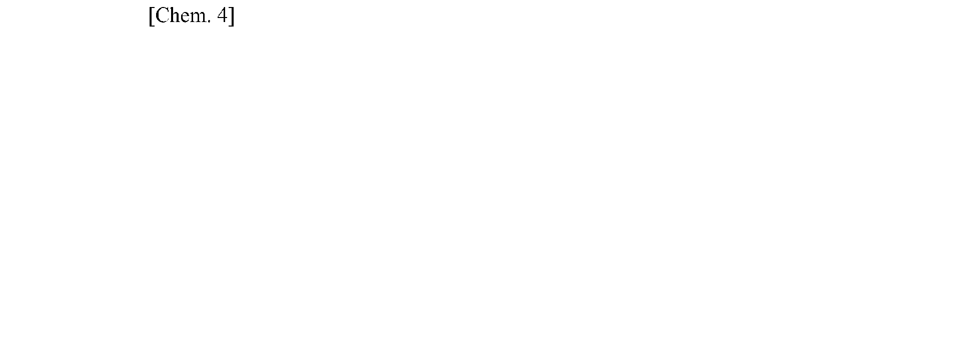 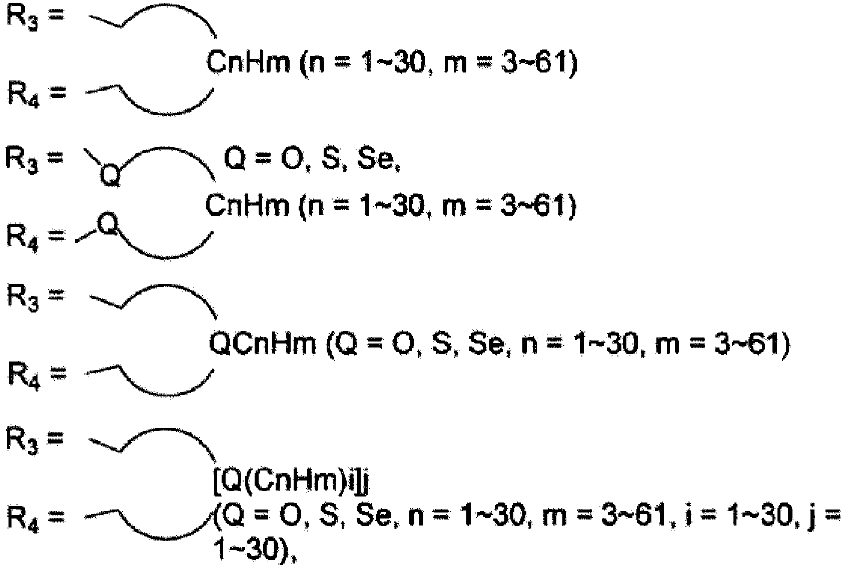 wherein R₁, R₂, R₃, R₄, and R' may be identical or different.
[2] The single crystalline thin film [1] of the molecular metal of the present invention is preferably a compound represented by a formula shown below: 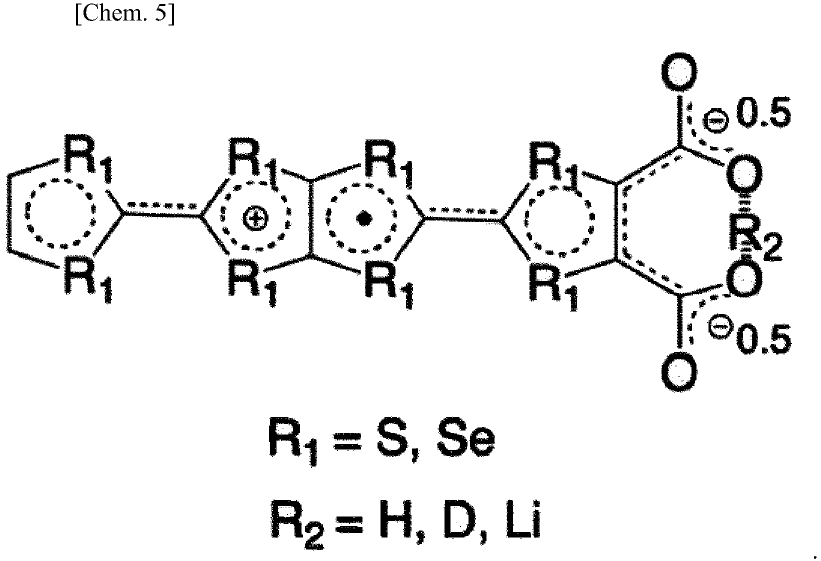
[3] The single crystalline thin films [1] and [2] of the molecular metal of the present invention preferably have a room temperature conductivity measured using a two-terminal method of 1 × 10⁻³ to 1 × 10⁷ S/cm, more preferably 1 to 1 × 10⁶ S/cm, and still more preferably 1 × 10² to 1 × 10⁵ S/cm.
[4] The single crystalline thin films [1] to [3] of the molecular metal of the present invention preferably have a film thickness of 1 nm or more and 1000 nm or less.
[5] A method for manufacturing a single-component organic molecular metallic (molecular metal) single crystalline thin film of the present invention, which is based on the contents of P1 or P3, comprises the steps of
   adding an aqueous alkali metal hydroxide solution at a third ratio to a single-component organic molecular metallic single crystal having a predetermined shape under a temperature condition of -10°C to 100°C;
   leaving the aqueous alkali metal hydroxide solution to stand to prepare a single crystalline thin film aqueous solution;
   forming a single crystalline thin film using the single crystalline thin film aqueous solution; and
   washing the formed single crystalline thin film.
[6] In the manufacturing method [5] of the present invention, the temperature condition in the step of adding the aqueous alkali metal hydroxide solution at the third ratio to the single-component organic molecular metallic single crystal having the predetermined shape is preferably a temperature condition of 0°C to 50°C.
[7] In the manufacturing method [5] or [6] of the present invention, the temperature condition in the step of leaving the aqueous alkali metal hydroxide solution to stand is preferably a temperature condition of -10°C to 100°C, and more preferably a temperature condition of 0°C to 50°C.
[8] In any one of the manufacturing methods [5] to [7] of the present invention, the temperature condition in the step of forming the single crystalline thin film using the single crystalline thin film aqueous solution is preferably a temperature condition of -10°C to 100°C, and more preferably a temperature condition of 0°C to 50°C.
[9] In any one of the manufacturing methods [5] to [8] of the present invention, the temperature condition in the step of washing the formed single crystalline thin film is preferably a temperature condition of -10°C to 100°C, and more preferably a temperature condition of 0°C to 50°C.
[10] In any one of the manufacturing methods [5] to [9] of the present invention, the step of forming the single crystalline thin film using the single crystalline thin film aqueous solution preferably includes any one of a drop casting method, a spin coating method, a dip coating method, a spray coating method, a Langmuir-Blodgett film formation method, and a surface functionalization method for substrates and particles.
[11] In any one of the manufacturing methods [5] to [10] of the present invention, the aqueous alkali metal hydroxide solution preferably contains any one of an aqueous lithium hydroxide solution, an aqueous sodium hydroxide solution, and an aqueous potassium hydroxide solution.
[12] In the manufacturing method [11] of the present invention, the aqueous alkali metal hydroxide solution preferably has a pH of 7.5 or more and 12 or less.
[13] In the manufacturing method [5] of the present invention, the third ratio is preferably 1 × 10⁻³ times or more and 1 × 10¹⁰ times or less, more preferably 2 times or more and 1 × 10⁸ times or less, and most preferably 2 times or more and 1 × 10⁶ times or less.
[14] In the manufacturing methods [5] to [13] of the present invention, the single-component organic molecular metallic single crystal is preferably obtained by pulverizing an organic metal molecular powder exhibiting high conductivity, adding a high-boiling-point organic solvent having a boiling point of 100°C to 250°C and a basic additive having a pH of 7.5 or more to a weight of the organic metal molecular powder, and then leaving a mixture obtained to stand under a temperature condition of 0°C to 50°C to obtain a brownish needle-like single crystal of fused tetrathiafulvalene-extended dicarboxylate.
[15] In the manufacturing method [14] of the present invention, an organic metal molecule is preferably a molecule that is chemically stabilized by electrically neutralizing a radical cation on a fused tetrathiafulvalene skeleton or a fused tetraselenafulvalene skeleton by a proton defect.
[16] In the manufacturing method [15] of the present invention, the organic metal molecule is preferably fused tetrathiafulvalene-extended dicarboxylate (TED for short) or fused tetraselenafulvalene dicarboxylate that is a selenium-substituted product thereof.
[17] In the manufacturing method [16] of the present invention, the fused tetrathiafulvalene-extended dicarboxylate powder or the fused tetraselenafulvalene dicarboxylate is preferably manufactured by a step represented by a chemical formula shown below: 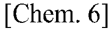 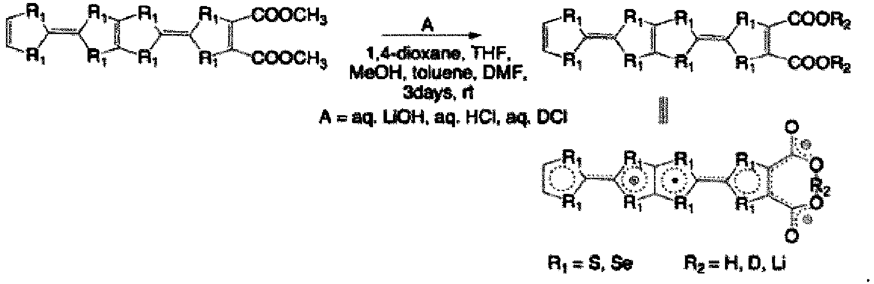
[18] The single crystalline thin films [1] to [4] of the molecular metal of the present invention preferably have a room temperature conductivity measured using a four-terminal method of 1 × 10 to 1 × 10¹² S/cm, more preferably 1 × 10² to 1 × 10¹⁰ S/cm, and still more preferably 1 × 10³ to 1 × 10⁹ S/cm.

The single-component organic molecular metallic (molecular metal) single crystalline thin film (single crystalline TED thin film) has anisotropy, and it is preferable that at least the conductivity in either the a-axis direction or the b-axis direction is within the above-mentioned range. In addition, it is also possible that the conductivity in both the directions is within the above-mentioned range.

Further, solutions to the problems based on the contents of P2 are as follows.
[1] A single crystalline thin film of a synthetic metal (molecular metal), preferably having a transmittance of light with a wavelength of 360 to 830 nm of 80% or more,
   wherein the molecular metal is a compound represented by Formula (I) shown below: 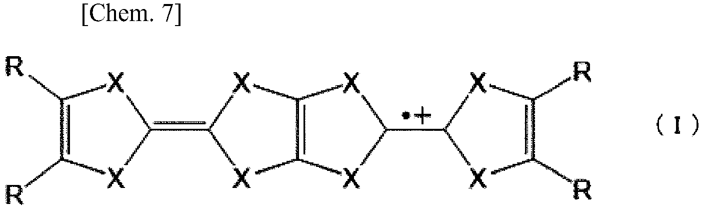
   wherein X represents at least one atom selected from the group consisting of S, O, and Se, and a plurality of Xs may be identical or different, and
   R represents a hydrogen atom or a monovalent substituent, and a plurality of Rs may be identical or different, provided that at least one of Rs is at least one group selected from the group consisting of groups represented by Formula (II) shown below, and Rs substituted with adjacent atoms may be linked to each other to form a ring: 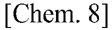 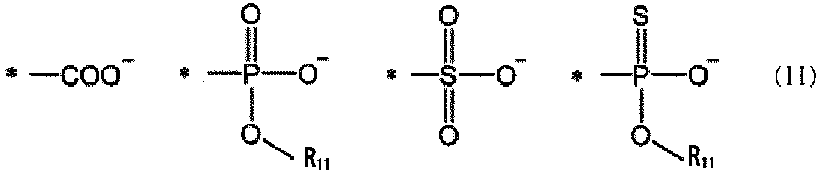
   wherein * represents a bonding position, and R₁₁ represents a hydrogen atom or an alkyl group having 1 to 4 carbon atoms.
[2] The single crystalline thin film according to [1], having a transmittance of light with a wavelength of 200 to 360 nm of 60% or more.
[3] The single crystalline thin film according to [1] of [2], having a film thickness of 1 to 20 nm.
[4] The single crystalline thin film according to any one of [1] to [3], comprising a mono- to deca-molecular layer of the compound represented by Formula (I).
[5] The single crystalline thin film according to any one of [1] to [4], wherein the molecular metal is a compound represented by Formula (III) shown below: 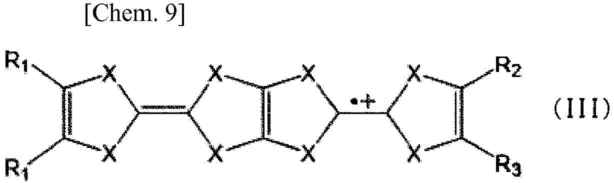
   wherein X represents an atom of S or Se, and a plurality of Xs may be identical or different,
   R₁ represents a hydrogen atom, a hydrocarbon group, a (poly)alkyleneoxy group, or a (poly)alkylenethio group, and two Ris may be identical or different, and
   either of R₂ and R₃ is a group represented by Formula (II), and the other is a Bronsted acid group corresponding to the group represented by Formula (II), or a salt thereof.
[6] The single crystalline thin film according to any one of [1] to [5], wherein the molecular metal is a compound represented by Formula (IV) shown below: 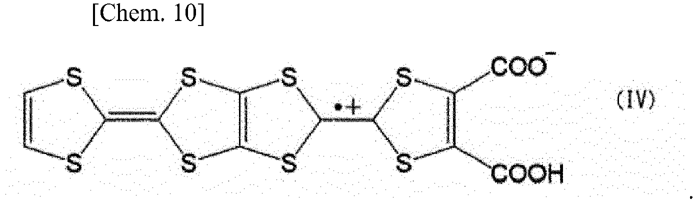
[7] The single crystalline thin film according to any one of [1] to [6], having an electrical conductivity according to evaluation by a two-terminal method of 1 × 10⁻³ to 1 × 10⁷ S/cm.
[8] A component comprising the single crystalline thin film according to any one of [1] to [7],
   the component being an optical component or an electronic component.
[9] The component according to [8], which is a transparent electrode or an optical fiber.
[10] A method for manufacturing the single crystalline thin film according to any one of [1] to [7], the method comprising:
   preparing a mixed liquid containing a single crystal of the molecular metal that is the compound represented by Formula (I) and an aqueous alkali metal hydroxide solution;
   leaving the mixed liquid to stand for a predetermined time;
   bringing a base material into contact with the mixed liquid after being left to stand to form the single crystalline thin film on the base material; and
   washing the single crystalline thin film formed on the base material.
[11] The manufacturing method according to [10], wherein the predetermined time for leaving the mixed liquid to stand is 168 hours or more.
[12] The manufacturing method according to [10] or [11], wherein in the mixed liquid, a weight ratio of the aqueous alkali metal hydroxide solution to the single crystal of the molecular metal is 2 to 1 × 10⁶.
[13] The manufacturing method according to any one of [10] to [12], wherein the aqueous alkali metal hydroxide solution has a pH of 7.5 to 11.
[14] The manufacturing method according to any one of [10] to [13], wherein by leaving of the mixed liquid to stand, in the mixed liquid, a synthetic metal piece as a monomolecular layer, crystallinity of the monomolecular layer in a two-dimensional direction being maintained, is exfoliated from the single crystal of the molecular metal.

Further, the solutions to the problems based on the contents of P2 are described as follows.

According to a first aspect of the present invention, which is based on the contents of P2, there is provided a single crystalline thin film of a synthetic metal (molecular metal), having a transmittance of light with a wavelength of 360 to 830 nm of 80% or more, wherein the molecular metal is a compound represented by Formula (I) described later.

The single crystalline thin film may have a transmittance of light with a wavelength of 200 to 360 nm of 60% or more. The single crystalline thin film may have a film thickness of about 1 to 20 nm. The single crystalline thin film may include an approximately mono- to deca-molecular layer of the compound represented by Formula (I).

The molecular metal may be a compound represented by Formula (III) described later or a compound represented by Formula (IV) described later.

The single crystalline thin film may have an electrical conductivity according to evaluation by a two-terminal method of 1 × 10⁻³ to 1 × 10⁷ S/cm.

According to a second aspect of the present invention, which is based on the contents of P2, there is provided a component comprising the single crystalline thin film according to the first aspect,
the component being an optical component or an electronic component.

The component may be a transparent electrode or an optical fiber.

According to a third aspect of the present invention, which is based on the contents of P2, there is provided a method for manufacturing the single crystalline thin film according to the first aspect, the method comprising: preparing a mixed liquid containing a single crystal of the molecular metal that is the compound represented by Formula (I) and an aqueous alkali metal hydroxide solution; leaving the mixed liquid to stand for a predetermined time; bringing a base material into contact with the mixed liquid after being left to stand to form the single crystalline thin film on the base material; and washing the single crystalline thin film formed on the base material.

The predetermined time for leaving the mixed liquid to stand may be 168 hours or more. In the mixed liquid, a weight ratio of the aqueous alkali metal hydroxide solution to the single crystal of the molecular metal may be 2 to 1 × 10⁶. The aqueous alkali metal hydroxide solution may have a pH of 7.5 to 11. In addition, by leaving of the mixed liquid to stand, in the mixed liquid, a synthetic metal piece as a monomolecular layer, crystallinity of the monomolecular layer in a two-dimensional direction being maintained, may be exfoliated from the single crystal of the molecular metal.

### ADVANTAGEOUS EFFECTS OF INVENTION

With use of the single-component organic molecular metallic (molecular metal) single crystalline thin film of the present invention, which is based on the contents of P1 or P3, it is possible to make a thin film having a nanoscale thickness, so that the room temperature electrical conductivity is exponentially improved.

With use of the method for manufacturing a single-component organic molecular metallic (molecular metal) single crystalline thin film of the present invention, which is based on the contents of P1 or P3, it is possible to make a thin film suitable for intended use, and the method has a very large spreading effect in many electronic equipment including electrodes.

The single crystalline thin film of a synthetic metal (molecular metal) of the present invention, which is based on the contents of P2, has high electrical conductivity, high light transmissivity, and ultra-thin film properties.

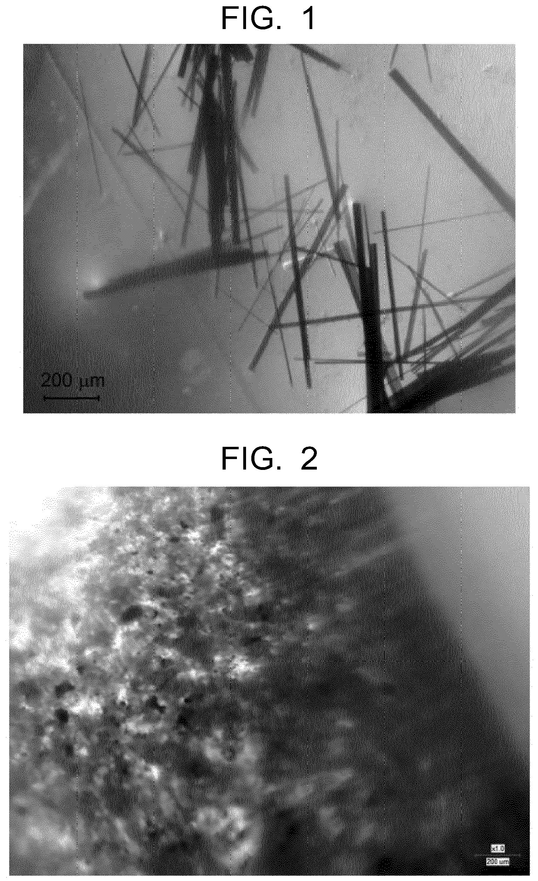

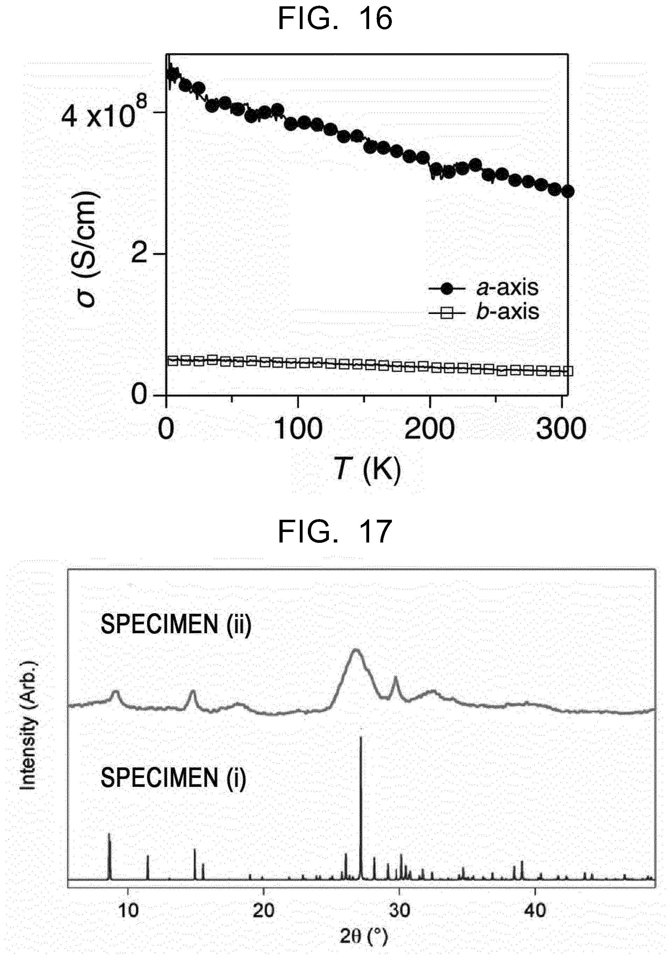

In the following, drawings based on the contents of P1 or P3 are shown in FIGS. 1 to 16. Among these drawings, for drawings identical with drawings based on the contents of P2, a description to that effect is given. Furthermore, among the drawings based on the contents of P2, drawings that do not overlap with FIGS. 1 to 16 are shown in FIGS. 17 to 21. These drawings will be briefly described.

### BRIEF DESCRIPTION OF DRAWINGS

FIG. 1 is a photograph showing an example of an organic metal (TED) needle-like single crystal grown by a manufacturing method of the present invention. It is an optical micrograph of a TED (Tetrathiafulvalene-Extended Dicarboxylate) single crystal (specimen (i)) produced in the examples (same as FIG. 1 of P2).
FIG. 2 is a photograph outside the scope of the manufacturing method of the present invention, and shows a comparative example in which an organic metal (TED) needle-like single crystal was not obtained. It is an optical micrograph of amorphous TED (specimen (ii)) produced in the section of EXAMPLES (same as FIG. 1 of P2).
FIG. 3A shows a unit cell structure in TED single crystal structure analysis (unit: angstrom). The numbers in parentheses indicate the error of the last digit (same as FIG. 4A of P2).
FIG. 3B shows a b-axis projection of a layered structure in the TED single crystal structure analysis (same as FIG. 4B of P2).
FIG. 3C shows an a-axis projection of a layered structure in the TED single crystal structure analysis (same as FIG. 4C of P2).
FIG. 3D shows an arrangement of π orbitals between molecules in the TED single crystal structure analysis (same as FIG. 4D of P2).
FIG. 4A is a micrograph showing a measurement method in which four terminals of gold electrodes are attached to a TED single crystal.
FIG. 4B is a graph showing the temperature dependence of electrical resistance of the TED single crystal by a four-terminal method using the gold electrodes shown in FIG. 4A.
FIG. 5 is a graph showing the reflectance of the TED single crystal at room temperature.
FIG. 6 is a graph showing powder X-ray diffraction patterns of an organic metal (TED) needle-like single crystal (lower part) and an organic metal (TED) powder (upper part) at room temperature.
FIG. 7A is an AFM image of a TED monomolecular layer film having a film thickness measured in a tapping mode of about 1.6 nm, which shows an example of the present invention.
FIG. 7B is an AFM image of a TED thin film having a film thickness measured in a tapping mode of about 4 nm, which shows an example of the present invention.
FIG. 7C is a diagram showing a result of film thickness measurement by tapping of a TED thin film having a film thickness of about 5 nm, which shows an example of the present invention.
FIG. 7D is a graph showing a correlation between the film thickness and the number of layers (1 to 10 layers). The shaded portion indicates an error bar, which results from variation in sample quality, a measurement error, or the like (same as FIG. 5A of P2).
FIG. 7E is a graph showing a correlation between the film thickness and the number of layers (1 to 500 layers) (same as FIG. 5B of P2).
FIG. 8A is a drawing showing an arrangement of a sample and electrodes in a two-terminal method used in the conductivity measurement of the present invention.
FIG. 8B is a drawing showing an arrangement of a sample and electrodes in a four-terminal method.
FIG. 9A is an optical microscope image of a TED monomolecular layer film having a film thickness of about 1.6 nm and having a terminal of a silver electrode attached to both the two ends, which shows an example of the present invention.
FIG. 9B is an optical microscope image of a TED thin film having a film thickness of about 8 nm and having a terminal of a silver electrode attached to both the two ends, which shows an example of the present invention.
FIG. 9C is an optical microscope image of a TED thin film having a film thickness of about 145 nm and having a terminal of a silver electrode attached to both the two ends, which shows an example of the present invention.
FIG. 10A shows room temperature current-voltage (IV) characteristics of a TED monomolecular layer film having a film thickness of about 1.6 nm and having a terminal of a silver electrode attached to both the two ends, which shows an example of the present invention (same as FIG. 6A of P2).
FIG. 10B shows room temperature current-voltage (IV) characteristics of a TED thin film having a film thickness of about 8 nm and having a terminal of a silver electrode attached to both the two ends, which shows an example of the present invention (same as FIG. 6B of P2).
FIG. 10C shows room temperature current-voltage (IV) characteristics of a TED thin film having a film thickness of about 145 nm and having a terminal of a silver electrode attached to both the two ends, which shows an example of the present invention (same as FIG. 6C of P2).
FIG. 11 is a graph showing a relationship between film thickness and room-temperature conductivity measured by a two-terminal method, which shows examples of the present invention (the plot is same as that in FIG. 7 of P2).
FIG. 12A shows the temperature dependence of resistivity of a TED monomolecular layer film having a film thickness of about 1.6 nm and having a terminal of a silver electrode attached to both the two ends, which shows an example of the present invention (same as FIG. 8A ofP2).
FIG. 12B shows the temperature dependence of resistivity of a TED thin film having a film thickness of about 8 nm and having a terminal of a silver electrode attached to both the two ends, which shows an example of the present invention (same as FIG. 8B of P2).
FIG. 12C shows the temperature dependence of resistivity of a TED thin film having a film thickness of about 145 nm and having a terminal of a silver electrode attached to both the two ends, which shows an example of the present invention (same as FIG. 8C of P2).
FIG. 13 is an AFM image of a single crystalline TED monolayer film, which shows an example of the present invention.
FIG. 14 is an optical microscope image of a single crystalline TED monolayer film to which four terminals of gold electrodes are attached, which shows an example of the present invention (the monolayer film exists inside the dotted line).
FIG. 15 shows room temperature current-voltage (IV) characteristics in the a-axis and b-axis directions of a single crystalline TED monolayer film, which shows an example of the present invention.
FIG. 16 shows the temperature dependence of electrical conductivity in the a-axis and b-axis directions of a single crystalline TED monolayer film, which shows an example of the present invention.
FIG. 17 shows X-ray diffraction patterns of specimens (i) and (ii) produced in the examples (same as FIG. 3 of P2).
FIG. 18A is a graph showing visible light transmittance of TED single crystalline thin films (specimens S1 to S3) measured at room temperature in the examples.
FIG. 18B is a graph showing visible light reflectance of the TED single crystalline thin films (specimens S1 to S3) measured at room temperature in the examples.
FIG. 19A is a graph showing visible light transmittance of a TED single crystal (specimen (i)) measured at room temperature in the examples.
FIG. 19B is a graph showing visible light reflectance of the TED single crystal (specimen (i)) measured at room temperature in the examples.
FIG. 20A is a graph showing ultraviolet light transmittance of the TED single crystalline thin films (specimens S1 to S3) measured at room temperature in the examples.
FIG. 20B is a graph showing ultraviolet light reflectance of the TED single crystalline thin films (specimens S1 to S3) measured at room temperature in the examples.
FIG. 21 is a flowchart describing a method for manufacturing a single crystalline thin film of a molecular metal of an embodiment of the present invention.

### DESCRIPTION OF EMBODIMENTS

The description of the components described below may be made based on representative embodiments of the present invention, but the present invention is not limited to such embodiments. In the present description, a numerical range represented by "to" means a range including numerical values described before and after "to" as a lower limit value and an upper limit value. In addition, in the notation of a chemical group (atomic group) in the present description, the notation that does not describe substitution or unsubstitution includes those having a substituent as well as those having no substituent as long as the effect of the present invention is not impaired. For example, an "alkyl group" includes not only an alkyl group having no substituent (unsubstituted alkyl group) but also an alkyl group having a substituent (substituted alkyl group). This also applies to compounds.

### [Single crystalline thin film of synthetic metal (molecular metal)]

Hereinafter, the single crystalline thin film of an embodiment of the present invention will be described. The single crystalline thin film of an embodiment of the present invention is a single crystalline thin film of a compound represented by Formula (I) described later (hereinafter referred to as a "compound A"). The compound A is a derivative compound obtained by introducing a specific substituent into fulvalene such as tetrathiafulvalene or a fused ring compound thereof. In spite of being an organic compound, the compound A is an undoped synthetic metal that exhibits conductivity without the addition of a doping agent. The compound A tends to form a sheet structure in which molecules are two-dimensionally assembled by its extended π-conjugated part, and this tendency delocalizes electrons on zwitterionic radical species in the molecular array. As a result, the compound A has excellent conductivity. More specifically, it is presumed that the compound A is metallized by delocalization of molecular spins, which are stabilized by a proton defect contained in an intramolecular hydrogen bond, into a two-dimensional sheet that is self-assembled by intermolecular force.

The present inventors have succeeded in producing a single crystal of the compound A and further thinning the single crystal. Then, the present inventors have found that the single crystalline thin film has both high transmittance to deep ultraviolet light to visible light and high electrical conductivity. In addition, the single crystalline thin film of the compound A achieves an ultra-thin film thickness on a nanometer scale that cannot be achieved by an amorphous thin film, and further has exponentially improved electrical conductivity and visible light transmittance as compared with those of an amorphous thin film.

The single crystalline thin film of an embodiment of the present invention preferably includes a mono- to deca-molecular layer of the compound A, and more preferably includes a mono- to tri-molecular layer of the compound A, for example. In addition, the single crystalline thin film of an embodiment of the present invention preferably has a film thickness of 1 to 20 nm, and more preferably 1 to 10 nm, for example. When the configuration and/or thickness of the single crystalline thin film is within the above-mentioned ranges, high transmittance to deep ultraviolet light to visible light and high electrical conductivity are more easily realized.

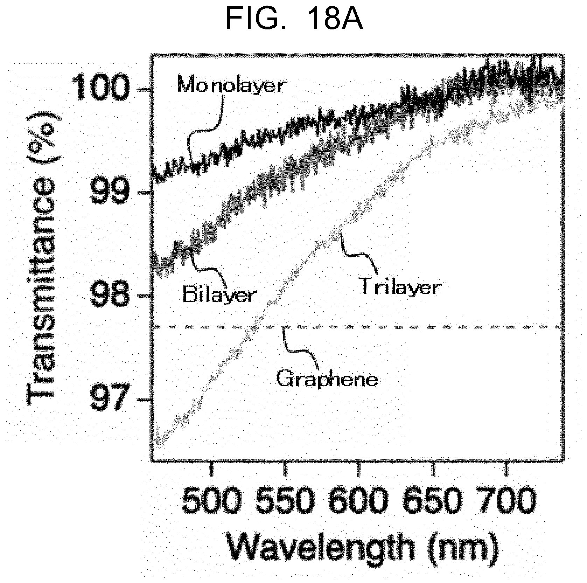

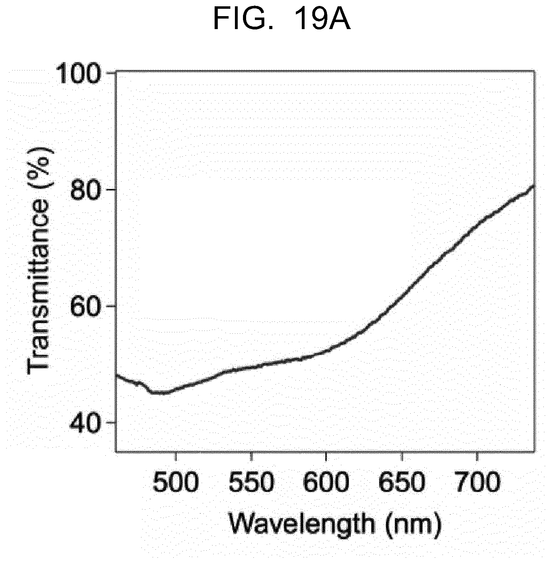

The single crystalline thin film may have a visible light (wavelength: 360 to 830 nm) transmittance of 80% or more, 90% or more, or 98% or more, for example. As shown in FIG. 19A, a single crystal of the compound A that is not a thin film (needle-like single crystal whose long axis is on a micrometer scale) has a large absorption band in the visible light region (around 490 nm), is colored in reddish brown, and has very low transparency. In contrast, as shown in FIG. 18A, single crystalline thin films of an embodiment of the present invention do not have a large absorption band in the visible light region, are almost colorless, and have high transparency. As can be understood from the comparison between FIGS. 18A and 19A, the light transmission spectrum of the crystal of the compound A is changed in shape due to thinning of the crystal. More specifically, in the single crystalline thin film of an embodiment of the present invention, the increase of light transmission amount is not simply due to the thin film thickness, but the light transmission characteristics are changed due to the quantum effect, whereby the high transmittance in the visible light region is realized.

The single crystalline thin film may have a deep ultraviolet light (wavelength: 200 to 360 nm) transmittance of 60% or more, 80% or more, or 90% or more, for example. Materials having high transmittance in the deep ultraviolet light (DUV) region are very limited, and reported materials are insulators with large bandgaps, such as oxides and fluorides of light metal elements, and silica glass. Meanwhile, the single crystalline thin film of an embodiment of the present invention has both high transmittance to deep ultraviolet light and high electrical conductivity.

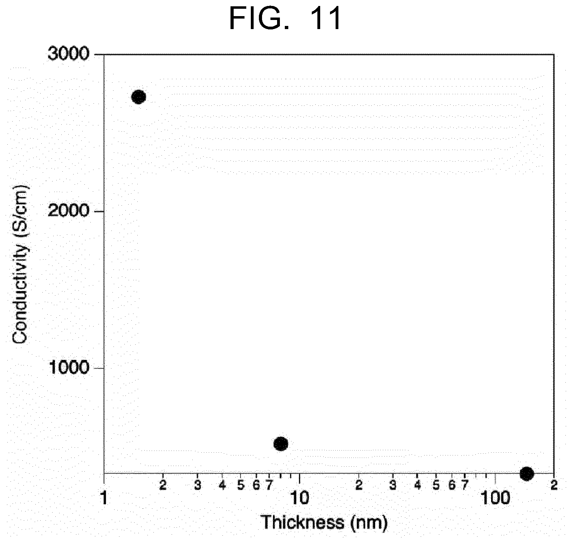

The single crystalline thin film of an embodiment of the present invention may have an electrical conductivity according to evaluation by a two-terminal method of 1 × 10⁻³ to 1 × 10⁷ S/cm, 1 to 1 × 10⁷ S/cm, or 1 × 10² to 1 × 10⁶ S/cm, for example. The single crystal of the compound A is improved in electrical conductivity due to thinning of the crystal, and as shown in FIG. 11, the thinner the film thickness is, the higher the electrical conductivity is. Since an external factor such as contamination of impurities is not observed in the analysis by photoelectron spectroscopy, this is considered to be due to a quantum effect on a band structure, which is found in graphene or the like and caused by confining electrons in a two-dimensional nanoscale space.

The single crystalline thin film of an embodiment of the present invention may be composed of only the compound A or may contain a crystallization solvent intercalated between sheet structures. In addition, the compound A is preferably composed of one type of the compound represented by Formula (I).

### [Compound A]

The synthetic metal (compound A) that is the molecular metal of an embodiment of the present invention is a compound represented by Formula (I) shown below or a salt thereof. The form of the salt formed by the compound A is not particularly limited, but it is preferably a form in which a Bronsted acid group described later (in a case where the compound A has a plurality of Bronsted acid groups, at least one of the Bronsted acid groups) forms a salt.

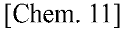

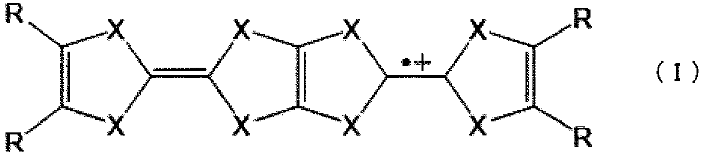

In Formula (I), X represents at least one atom selected from the group consisting of S, O, and Se, and a plurality of Xs may be identical or different, and

R represents a hydrogen atom or a monovalent substituent, and a plurality of Rs may be identical or different, provided that at least one of Rs is at least one group selected from the group consisting of groups represented by Formula (II) shown below, and Rs substituted with adjacent atoms may be linked to each other to form a ring.

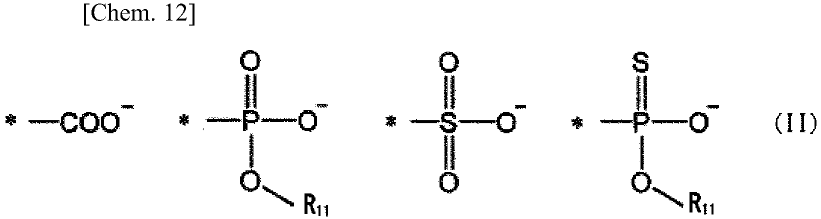

In Formula (II), * represents a bonding position, and R₁₁ represents a hydrogen atom or an alkyl group having 1 to 4 carbon atoms.

Here, among Rs in Formula (I), the monovalent substituent other than the group represented by Formula (II) (hereinafter, the substituent is referred to as a "substituent R‴) is not particularly limited, and examples thereof include a hydrogen atom, a hydrocarbon group (linear, branched, or cyclic), a (poly)alkyleneoxy group, and a (poly)alkylenethio group.

The number of carbon atoms in the hydrocarbon group is not particularly limited, but is generally preferably 1 to 30, and more preferably 1 to 20. The (poly)alkyleneoxy group is a group represented by Formula (i) shown below, and the number of carbon atoms in the alkylene chain moiety, R₁₂, is not particularly limited, but is generally preferably 1 to 30, and more preferably 1 to 20. The number of repetitions n1 of the alkyleneoxy unit is not particularly limited, but is generally preferably 1 to 30. The (poly)alkylenethio group is a group represented by Formula (ii) shown below, and the number of carbon atoms in the alkylene chain moiety, R₁₃, is not particularly limited, but is generally preferably 1 to 30, and more preferably 1 to 20. The number of repetitions n2 of the alkylenethio unit is not particularly limited, but is generally preferably 1 to 30. In Formulae (i) and (ii), * represents a bonding position.

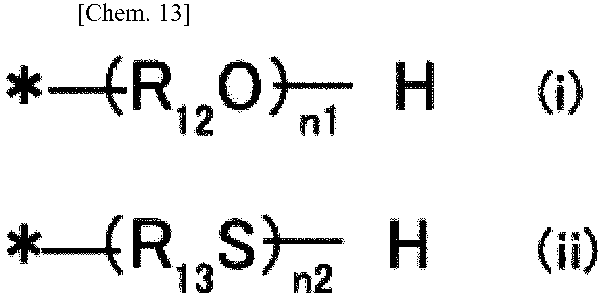

The group represented by Formula (II) is a conjugate base of a Bronsted acid group. The Bronsted acid group means a substituent having a function as a Bronsted acid, and means a network in which the compound A is self-assembled by hydrogen bonding (hydrogen bond network), or a substituent having a function of generating a proton defect in an intramolecular hydrogen bond.

Further, the monovalent substituent R' among Rs in Formula (I) may be a Bronsted acid group. Examples of the Bronsted acid group include a carboxy group, a sulfonic acid group, a phosphoric acid group, and a group represented by *-P(=X)OR₁₄OH (wherein X represents an oxygen atom or a sulfur atom, R₁₄ represents a hydrogen atom or an alkylene group having 1 to 4 carbon atoms, and * represents a bonding position).

The Bronsted acid group in the compound A may form a salt. In this case, the counter ion is not particularly limited, and examples thereof include an ammonium ion, an alkali metal ion (a lithium ion, a sodium ion, or a potassium ion), and an organic cation (a guanidinium ion, a pyridinium ion, an imidazolium ion, or an anilinium ion). The Bronsted acid group may form a hydroxylamine salt.

From the viewpoint that the compound A has more excellent stability, in Formula (I), it is preferable that one of Rs is a group represented by Formula (II), and the other one or more of Rs is a Bronsted acid group or a salt thereof. In this case, it is more preferable that one of Rs substituted with adjacent atoms is a group represented by Formula (II), and the other is a Bronsted acid group or a salt thereof.

Examples of a suitable form of the compound A include a compound represented by Formula (III).

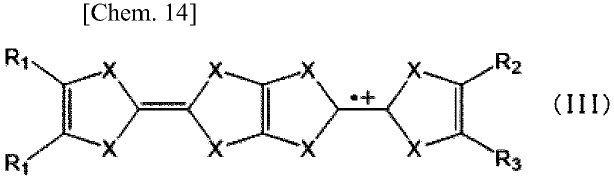

In Formula (III), X represents an atom of S or Se, and a plurality of Xs may be identical or different. R₁ is similar to the form of the monovalent substituent R' in formula (I), and two Ris may be identical or different. Either of R₂ and R₃ is a group represented by Formula (II), and the other is a Bronsted acid group corresponding to the group represented by Formula (II), or a salt thereof. Typically, a case can be mentioned where R₂ is *-COO⁻, and R₃ is *-COOH, *-COO⁻NH₄⁺, or *-COOLi (in each case, * represents a bonding position).

Examples of a further suitable form of the compound A include a compound represented by Formula (IV).

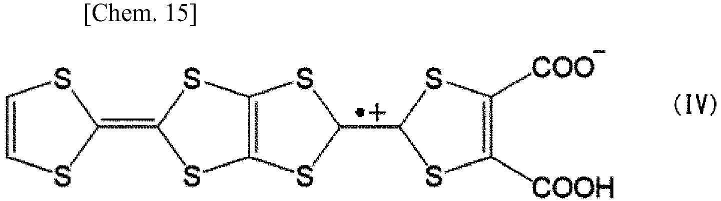

Hereinafter, the compound represented by Formula (IV) is also referred to as "TED" (Tetrathiafulvalene-Extended Dicarboxylate).

The method for synthesizing the compound A is not particularly limited, and a publicly known method can be applied. Examples of the method for synthesizing the compound A include the methods described in Patent Literatures 1 and 2.

### [Intended use of single crystalline thin film]

As described above, the single crystalline thin film of a synthetic metal (molecular metal) of an embodiment of the present invention has ultra-thin film properties, high electrical conductivity, and high light transmittance in the deep ultraviolet light to visible light region. Therefore, the single crystalline thin film can be applied to various devices such as optical components and electronic components. For example, the single crystalline thin film of a synthetic metal of an embodiment of the present invention is suitable for an electrode material of a transparent electrode. In addition, the single crystalline thin film of an embodiment of the present invention has high transmittance to deep ultraviolet light (DUV), and thus is suitable for a DUV transmissive material such as a DUV optical fiber.

Furthermore, the single crystalline thin film according to an embodiment of the present invention can be used in, for example, an electric wire, an information transmission medium, an electronic device, an electrode used in an electronic element, a spintronics element, an information communication element, a memory element, a magnetic shield, a medical magnetic shield, a magnet, a magnetic semiconductor, a field effect transistor (FET), a magnet-containing adhesive bandage, a head of a hard disk drive, a GMR (giant magnetoresistance effect) head for high-sensitivity reproduction, a solid magnetic memory, a magnetoresistive random access memory (MRAM), a fiber communication optical isolator, a material whose color changes by a magnetic field, and a material using an interaction between a conductive electron spin and an atomic magnetic moment.

In addition, the transparent electrode including the single crystalline thin film of an embodiment of the present invention can also be applied to, for example, a touch panel, a display device, a display, an electronic device, a liquid crystal display, a flat type television, a plasma display, an electronic ink, an anode (hole injection layer) in organic EL (electroluminescence), a solar cell, an antistatic agent, an electromagnetic wave shielding material, an optical coating agent, an infrared reflecting material, a gas sensor, an antireflection film, a surface treatment agent, a semiconductor laser, an optical device, an optical element, a bending resistant device, an electrolytic capacitor, electronic equipment, an electrode of a lithium ion battery, and a light emitting element.

### [Method for manufacturing single crystalline thin film]

The method for manufacturing a single crystalline thin film of an embodiment of the present invention is not particularly limited, but for example, the single crystalline thin film can be manufactured by the method described below.

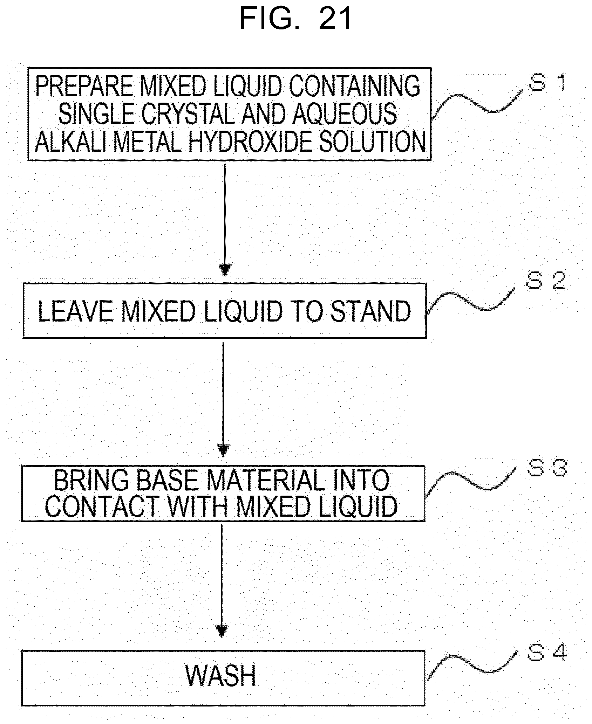

First, a mixed liquid containing (a) a single crystal of the compound A and (b) an aqueous alkali metal hydroxide solution is prepared (step S1 in FIG. 21), and then the prepared mixed liquid is left to stand for a predetermined time (step S2 in FIG. 21). The present inventors have found that through the above-mentioned steps, in the mixed liquid, a synthetic metal piece (two-dimensional crystal) is exfoliated in a layer form from the single crystal of the compound A. The synthetic metal piece exfoliated from the single crystal of the compound A, crystallinity of which in a two-dimensional direction is maintained, is an ultra-thin monomolecular layer. The reason (mechanism) why the synthetic metal piece is exfoliated from the single crystal of the compound A through the above-mentioned steps is not clear, but it is presumed that strong two-dimensionality of the bulk single crystal structure causes this phenomenon as can be seen in many examples in which graphene, transition metal dichalcogenide, or the like, which is composed of atomic (molecular) sheets that are assembled by weak intermolecular force (van der Waals force) and stacked in layers, is exfoliated into monolayer films. It is to be noted that this mechanism is based on a presumption, and does not limit the present invention at all.

### (a) Single crystal of compound A

The single crystal of the compound A may be manufactured by a publicly known method. For example, the single crystal of the compound A may be manufactured by the method described below, which is disclosed in Non-Patent Literature 2. First, the compound A is synthesized. The method for synthesizing the compound A is not particularly limited, and for example, the compound A may be synthesized by the method disclosed in Patent Literature 1 or 2. Then, a solution containing the synthesized powdery compound A (amorphous), a high-boiling-point organic solvent, and a basic additive is prepared. The prepared solution is left to stand for a predetermined time, whereby a needle-like single crystal of the compound A is precipitated in the solution. The precipitated single crystal is filtered, washed with water, and dried to obtain the single crystal of the compound A as a final product.

In the method for manufacturing the single crystal of the compound A, the high-boiling-point organic solvent is preferably a solvent having a boiling point of 100°C to 250°C, and examples thereof include dimethyl sulfoxide (DMSO), N,N-dimethylformamide (DMF), and N-methylpyrrolidone (NMP). These solvents may be used singly or in combination of two or more thereof. As the basic additive, an aqueous hydroxylamine solution, an aqueous ammonia solution, an aqueous dimethylamine solution, or the like can be used. These basic additives may be used singly or in combination of two or more thereof. The basic additive preferably has a pH of 7.5 or more. The ratio of the weight of the high-boiling-point organic solvent to the weight of the powdery compound A (amorphous) may be, for example, 30 to 400. The ratio of the weight of the basic additive to the weight of the powdery compound A (amorphous) may be, for example, 5 to 300.

### (b) Aqueous alkali metal hydroxide solution

As the aqueous alkali metal hydroxide solution, an aqueous solution of lithium hydroxide, sodium hydroxide, or potassium hydroxide is preferable, and an aqueous solution of lithium hydroxide is more preferable, from the viewpoint of promoting exfoliation of the compound A into monolayer films and reducing the manufacturing cost, for example. These aqueous solutions may be used singly or in combination of two or more thereof. The pH of the aqueous alkali metal hydroxide solution can be appropriately adjusted according to the amount of the compound A to be mixed with the aqueous alkali metal hydroxide solution, but from the viewpoint of promoting the exfoliation of the compound A into monolayer films, for example, the pH is preferably 7.5 to 11, and more preferably 9 to 11. The concentration of the alkali metal hydroxide in the aqueous alkali metal hydroxide solution is not particularly limited, and may be appropriately adjusted so that the aqueous solution may have a desired pH. Since the compound A is an acidic substance, the alkali metal hydroxide is neutralized by mixing with the compound A, and the pH of the aqueous solution decreases. The preferred pH range of the aqueous alkali metal hydroxide solution described above means a range of the pH before mixing with the compound A.

In the mixed liquid, the weight ratio of the aqueous alkali metal hydroxide solution to the single crystal of the compound A is not particularly limited and can be appropriately adjusted, but may be, for example, 2 to 1 × 10⁶ from the viewpoint of promoting the exfoliation of the compound A into monolayer films.

The time for leaving the prepared mixed liquid to stand (predetermined time) is not particularly limited, and may be, for example, 1 week (168 hours) or more, 1 week to 1 month, or 1 month to 6 months from the viewpoint of promoting the exfoliation of the compound A into monolayer films. The temperature of the mixed liquid during being left to stand is not particularly limited, and may be, for example, -10°C to 100°C, 0°C to 50°C, or room temperature.

Then, a base material is brought into contact with the mixed liquid after being left to stand to form the single crystalline thin film on the base material (step S3 in FIG. 21). The base material is not particularly limited, and can be appropriately selected according to the device to be used. For example, when the single crystalline thin film together with the base material are used in a transparent electrode or the like, the base material is preferably transparent to ultraviolet light and/or visible light (preferably has high transmittance). Examples of the base material include a glass substrate and a quartz substrate. The method for forming the single crystalline thin film (method for bringing the base material into contact with the mixed liquid) is not particularly limited, and the method can be appropriately selected from a drop casting method, a spin coating method, a dip coating method, a spray coating method, a Langmuir-Blodgett film formation method, a surface functionalization method for substrates and particles, and the like. Synthetic metal pieces exfoliated from the single crystal are dispersed in the mixed liquid after being left to stand. The synthetic metal pieces are deposited on a substrate to form a single crystalline thin film. Before bringing the mixed liquid into contact with the base material, a solvent such as water may be added to the mixed liquid to adjust the concentration of the synthetic metal pieces (or dilute the mixed liquid). The dilution makes it possible that the synthetic metal pieces are more likely to be uniformly dispersed in the mixed liquid. The temperature of the mixed liquid to be brought into contact with the base material is preferably around room temperature.

Then, the single crystalline thin film formed on the base material is washed (step S4 in FIG. 21). Washing removes unnecessary alkali components and the like attached to the single crystalline thin film. The washing method is not particularly limited, and examples thereof include a method of washing the single crystalline thin film disposed on the substrate with water.

As for the method for manufacturing the single crystalline thin film described above, the manufacturing process is simple, and the yield of a product including the single crystalline thin film can be increased. In addition, the film thickness of the single crystalline thin film can be adjusted, for example, by appropriately changing the manufacturing conditions of the manufacturing method described above. The manufacturing method is suitable, for example, for manufacturing an electrode using printing (manufacturing a printable electrode).

### EXAMPLES

Hereinafter, the present invention will be described in more detail with reference to examples. Materials, amounts used, proportions, contents of treatment, treatment procedures, and the like described in the following examples can be appropriately changed without departing from the gist of the present invention. Therefore, the scope of the present invention should not be restrictively interpreted by the following examples.

In the following examples, examples based on the contents of P1 or P3 will be described in detail, and then examples based on the contents of P2 will be described in detail.

### [I. Examples based on contents of P1 or P3]

### [Example 1]

Hereinafter, a method for growing a single crystalline thin film of fused tetrathiafulvalene-extended dicarboxylate (hereinafter also abbreviated as TED), an example of a single-component organic molecular metallic (molecular metal) single crystalline thin film, will be described.

### <Method for growing single crystal>

Dimethyl sulfoxide (DMSO) and hydroxylamine were purchased from Wako Pure Chemical Industries, Ltd. and Aldrich, respectively. TED was synthesized according to the following chemical formula showing a method for synthesizing a single-component organic molecular metal.

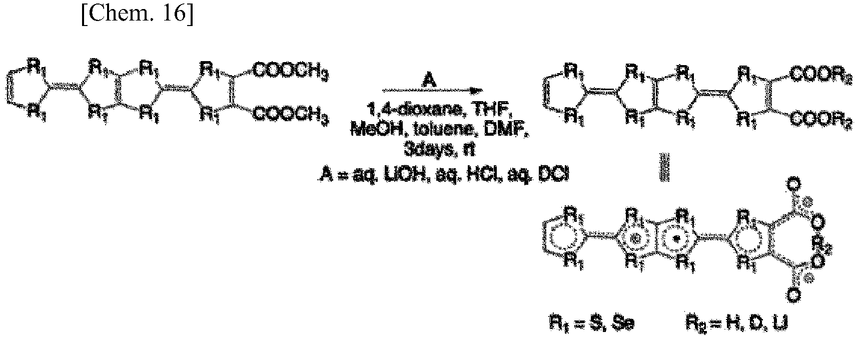

A TED powder crystal (1 mg) was pulverized in a mortar and dissolved in DMSO (160 µL). Hydroxylamine (a 50 wt% solution, 50 µL) was added to the solution to control the acidity of the solution. The solution was placed in a closed vial container and stored at room temperature for several months. The grown single crystal was collected by filtration, washed with water in an amount of about 10 times the weight of TED, and then naturally dried. A brownish needle-like single crystal is obtained with typical dimensions of 400 µm × 20 µm × 10 µm (see, for example, FIG. 1).

The TED powder is pulverized, a high-boiling-point organic solvent and a basic additive each at a certain ratio with respect to the weight of the TED powder are added, and then a mixture obtained is left to stand under a temperature condition of 0°C to 50°C for a long period of time to obtain a brownish needle-like single crystal. The grown single crystal is filtered, washed with an appropriate amount of water, and then dried to obtain a needle-like single crystal of TED.

### <Evaluation of physical properties of single crystals>

When the TED powder composed of nanoscale pieces is subjected to crystal growth by the method of Practical Example 1, a needle-like single crystal having a submilli-scale long axis is obtained (FIG. 1).

Table 1 shows an example of single crystal growth conditions in the present invention. The numerical values in the table indicate weight ratios of the used components with respect to the substrate weight taken as 1. Here, the solvents are, from the left, dimethyl sulfoxide (DMSO), dimethylformamide (DMF), N-methylpyrrolidone (NMP), hydroxylamine (NH₂OH), ammonia (NH₃), and dimethylamine (NH(Me)₂).

**[Table 1]**

| Example | Temperature(°C) | DMSO | DMF | NMP | NH₂OH | NH₃ | NH(Me)₂ |
|---|---|---|---|---|---|---|---|
| 1 | 25 | 0 | 169 | 0 | 0 | 67 | 0 |
| 2 | 25 | 145 | 0 | 0 | 45 | 0 | 0 |
| 3 | 25 | 0 | 169 | 0 | 54 | 0 | 0 |
| 4 | 25 | 145 | 0 | 0 | 45 | 0 | 0 |
| 5 | 25 | 364 | 0 | 0 | 214 | 0 | 0 |
| 6 | 25 | 145 | 0 | 0 | 134 | 0 | 0 |
| 7 | 25 | 145 | 0 | 0 | 89 | 0 | 0 |
| a | 25 | 91 | 0 | 0 | 179 | 0 | 0 |
| 9 | 25 | 91 | 0 | 0 | 9 | 0 | 0 |
| 10 | 25 | 218 | 0 | 0 | 67 | 0 | 0 |
| 11 | 25 | 145 | 0 | 0 | 45 | 0 | 0 |
| 12 | 25 | 73 | 0 | 0 | 22 | 0 | 0 |
| 13 | 25 | 145 | 0 | 0 | 45 | 0 | 0 |
| 14 | 25 | 36 | 0 | 0 | 11 | 0 | 0 |
| 15 | 25 | 131 | 17 | 0 | 54 | 0 | 0 |
| 16 | 25 | 15 | 152 | 0 | 54 | 0 | 0 |
| 17 | 25 | 73 | 85 | 0 | 54 | 0 | 0 |
| 18 | 25 | 116 | 34 | 0 | 54 | 0 | 67 |
| 19 | 25 | 29 | 136 | 0 | 54 | 0 | 0 |
| 20 | 25 | 136 | 0 | 29 | 54 | 0 | 0 |

### Example

### Temperature

Meanwhile, when the TED powder is not subjected to growth under the suitable conditions provided in the present invention (see, for example, Table 1), no single crystal grows and the powder remains as it is (FIG. 2).

In X-ray crystallography, the needle-like single crystal is determined to have a periodic structure in which two molecules are contained in an asymmetric unit, and have a layered crystal structure including molecular sheets stacked in the a-axis and b-axis directions.

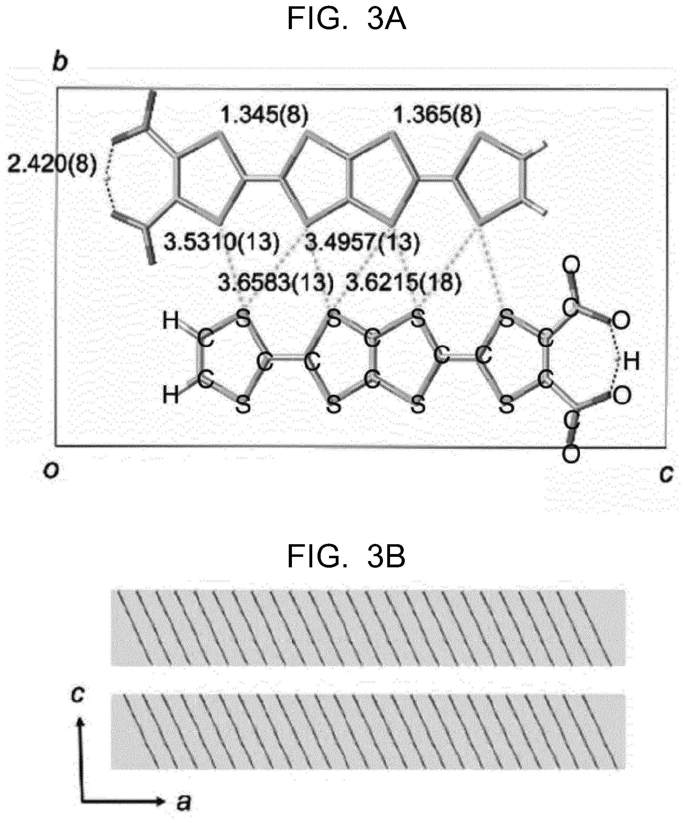

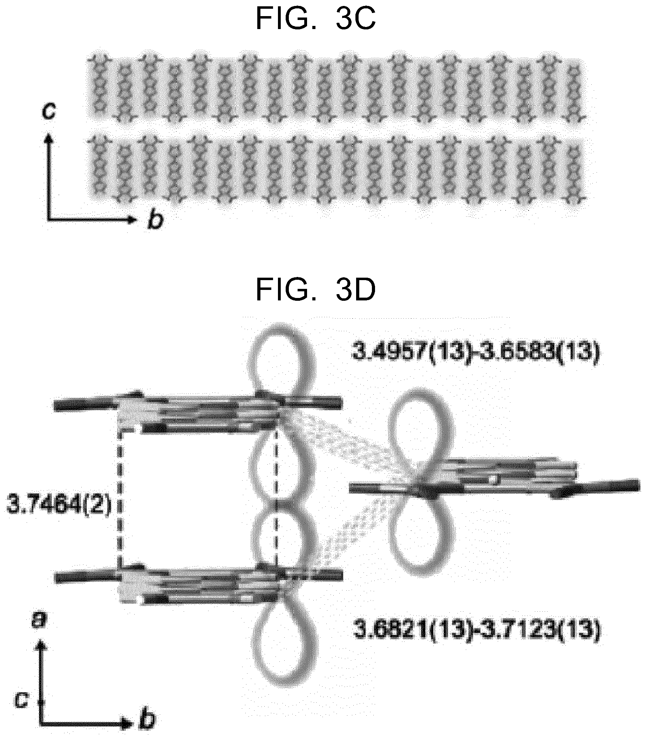

FIG. 3A shows a unit cell structure in TED single crystal structure analysis (unit: angstrom). The numbers in parentheses indicate the error of the last digit. FIG. 3B shows a b-axis projection of a layered structure in the TED single crystal structure analysis. FIG. 3C shows an a-axis projection of a layered structure in the TED single crystal structure analysis. FIG. 3D shows an arrangement of π orbitals between molecules in the TED single crystal structure analysis.

The crystal lattice parameters are as follows: P2₁/m, a = 3.7464(2) Å, b = 11.8946(6) Å, c = 20.2094(11) Å, b = 93.506(2) Å, V = 898.88(8) Å, R₁ = 5.38% (the X-ray structural analysis was performed at 113 K). The numerical values in parentheses indicate the error of the last digit in the X-ray structural analysis.

In FIG. 3A, the gray dotted lines and the black dotted line indicate the intermolecular distance of S ... S contacts given in angstrom and an intramolecular hydrogen bond, respectively. In FIGS. 3B and 3C, each gray shaded portion indicates a single molecular layer. In FIG. 3D, as for the overlapping modes of the π orbitals, the π orbitals are vertically overlapped by π ... π stacking along the a-axis and laterally overlapped by an S ... S contact along the b-axis.

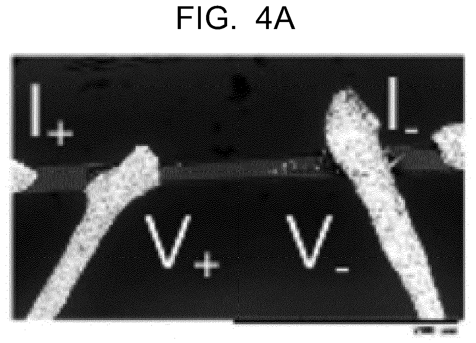

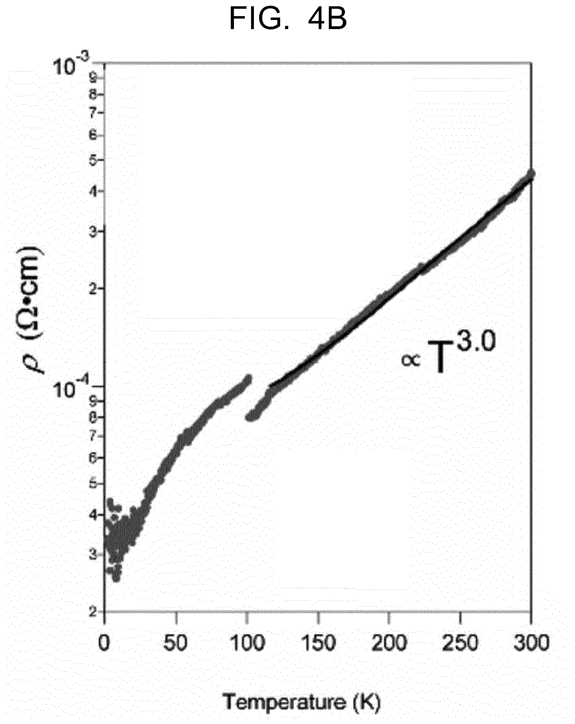

FIG. 4 show a micrograph and a graph showing the temperature dependence of electrical resistance of the TED single crystal by a four-terminal method using gold electrodes. FIG. 4A is an enlarged microscope image of gold electrode contact sites of the TED single crystal by the four-terminal method, and FIG. 4B is a graph showing the temperature dependence of electrical resistance of the TED single crystal. The electrical resistance ρ is proportional to the cube of the absolute temperature T. The electrical resistance ρ is about 5 × 10⁻³ [Ω·cm] at 300 K that is normal temperature, about 8 to 12 × 10⁻⁴ [Ω·cm] at around 113 K that is the boiling point temperature of liquefied natural gas LNG, about 8 × 10⁻⁴ [Ω·cm] at 77 K that is the boiling point temperature of liquid nitrogen, and about 2.5 to 5 × 10⁻⁴ [Ω·cm] at around 4 K that is the boiling point temperature of liquid helium.

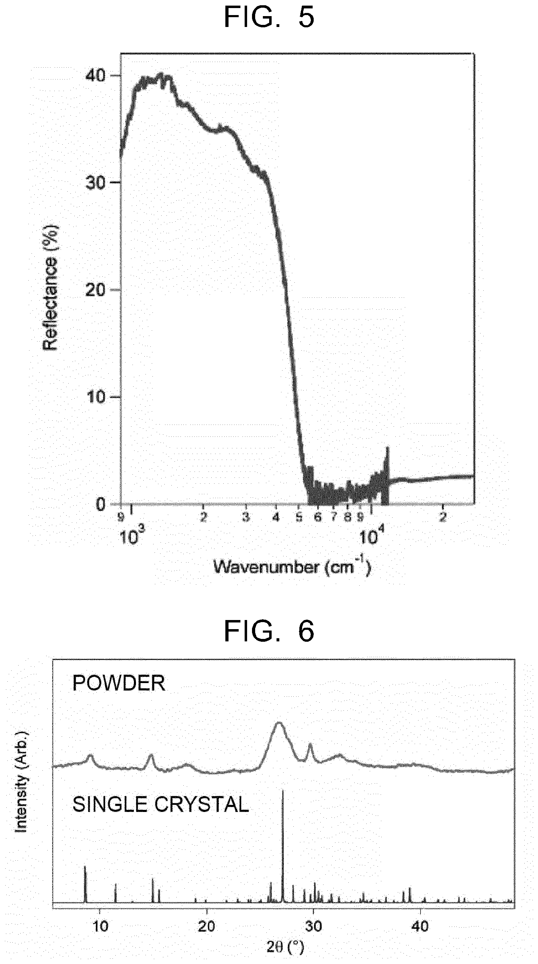

FIG. 5 is a graph showing the reflectance of the TED single crystal at room temperature. The reflectance is 30 to 40% in the wavenumber range of 1 to 4 × 10³ [cm⁻¹] corresponding to far infrared rays to middle infrared rays, whereas the reflectance is 0 to 2% in the wavenumber range of 4 to 12 × 10³ [cm⁻¹] included in the range of near infrared rays.

FIG. 6 is a graph showing powder X-ray diffraction patterns of an organic metal (TED) needle-like single crystal (lower part) and an organic metal (TED) powder (upper part) at room temperature. The single crystal has a sharp peak around a 2θ of 27°. Meanwhile, the powder has a high peak around a region of 25° to 28°.

### <X-ray crystallography>

Crystal structure analysis of the single crystal was performed using 1922 reflections in high resolution diffraction images collected at 113 K using a Rigaku Saturn CCD system. All atoms except hydrogen atoms were determined anisotropically.

### [Practical Example 2]

### <Method for preparing single crystalline thin film>

Then, an aqueous lithium hydroxide solution (0.5 µL) at a temperature of 0°C to 50°C and adjusted to a pH of 7.5 to 12 as an aqueous alkali metal hydroxide solution is added to one single crystal (approximately 400 × 20 × 10 µm in size) at room temperature.

The resulting solution is left to stand for at least 1 week.

Thereafter, a large excess (50 µL) of water is added to the solution, and the resulting mixture is left to stand for 10 minutes.

The TED single crystalline thin film aqueous solution was drop-cast on a glass substrate, then naturally dried, washed twice with water (50 µL) heated to 50°C, and then naturally dried for 20 minutes to obtain a desired metal thin film.

The film thickness was evaluated by measurement using a laser microscope or using an atomic force microscope (AFM).

### <Evaluation of film thickness>

The film thickness of the single crystalline thin film was evaluated using an atomic force microscope (AFM) (Bruker model MultiMode 8) and a silicon cantilever (SCANASYST-AIR) or Nano Search Microscope (SFT-3500 manufactured by Shimadzu Corporation) in the air. The surface state of three types of nanoscale thin films having different film thicknesses produced by the method of Practical Example 2 was observed using AFM measurement. The film thickness was determined using a tapping mode. It was recognized that various nanoscale single crystalline thin films including a monomolecular layer film (about 2 nm-thick) were obtained.

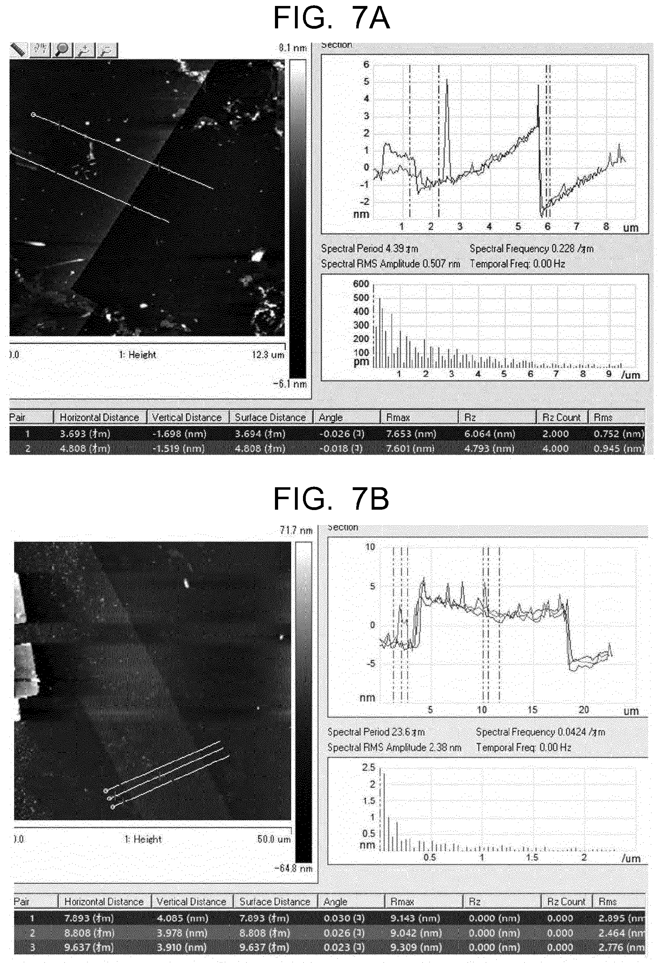

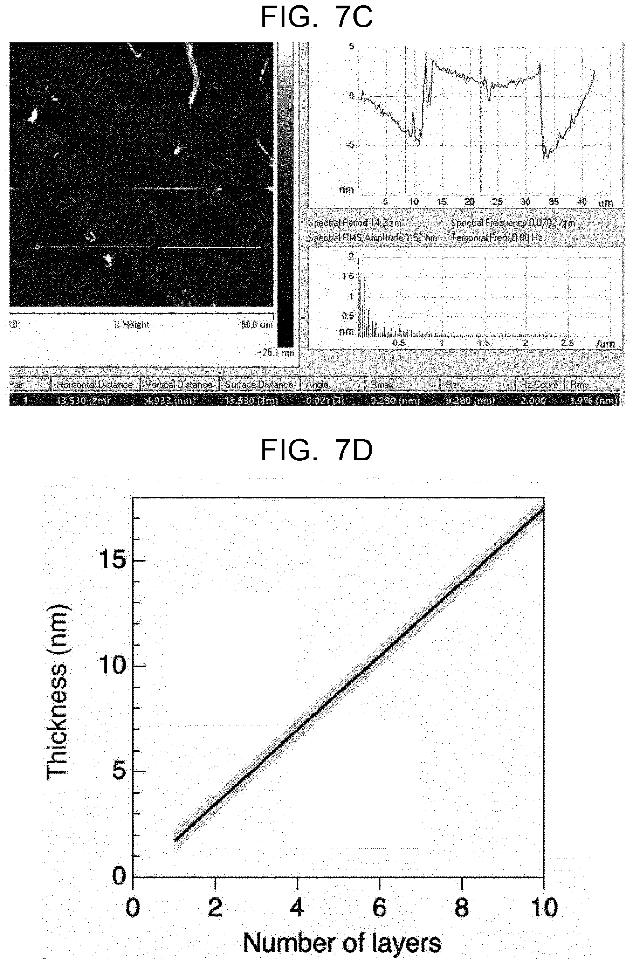

FIG. 7A is an AFM image of a TED monomolecular layer film having a film thickness measured in a tapping mode of 1 to 3 nm, which shows an example of the present invention. The image on the left side shows a scanning region of a probe, the upper right side shows a height profile diagram, and the lower right side shows a histogram. The lower table shows numerical data on a scanning line of the probe. FIG. 7B is an AFM image of a TED thin film having a film thickness of about 4 nm. FIG. 7C is an AFM image of a TED thin film having a film thickness of about 5 nm.

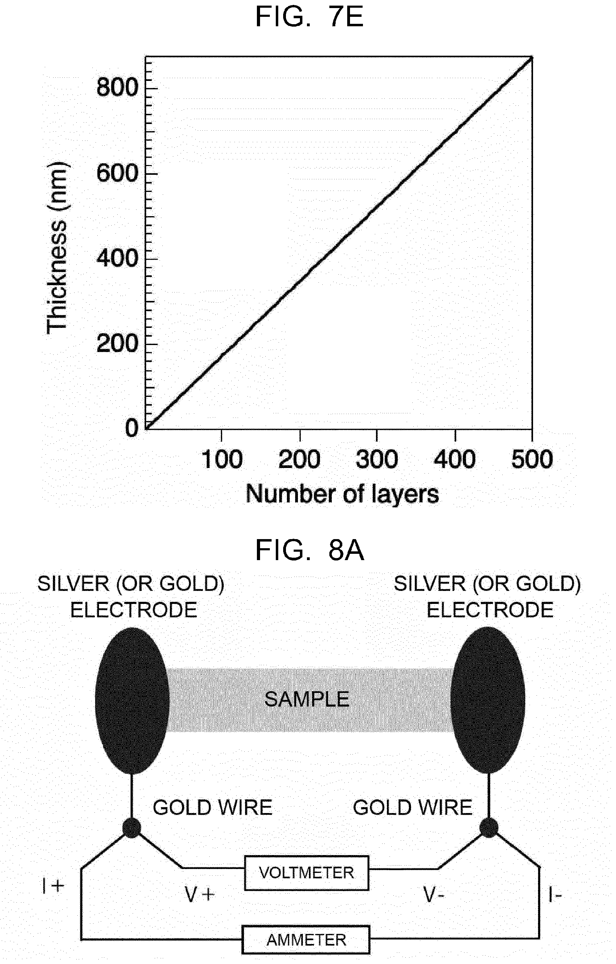

FIG. 7D is a graph showing a correlation between the film thickness and the number of layers (1 to 10 layers). The shaded portion indicates an error bar, which results from variation in sample quality, a measurement error, or the like. FIG. 7E is a graph showing a correlation between the film thickness and the number of layers (1 to 500 layers).

### <Evaluation of I-V characteristics>

FIG. 8A is a drawing showing an arrangement of a sample and electrodes in a two-terminal method used in the conductivity measurement of the present invention. In the drawing, a silver electrode or a gold electrode is provided as a positive or negative electrode terminal at both ends of the sample to be measured. A positive electrode of the potential and a positive electrode of the current are connected to the silver electrode as the positive electrode. The resistance value measured by the two-terminal method reflects the contribution of both the sample itself and the contact resistance arising from a junction between the sample and the silver electrode, but the two-terminal method is a sufficiently reliable method for the purpose of estimating the approximate resistance value of the sample and determining the temperature dependence of resistance.

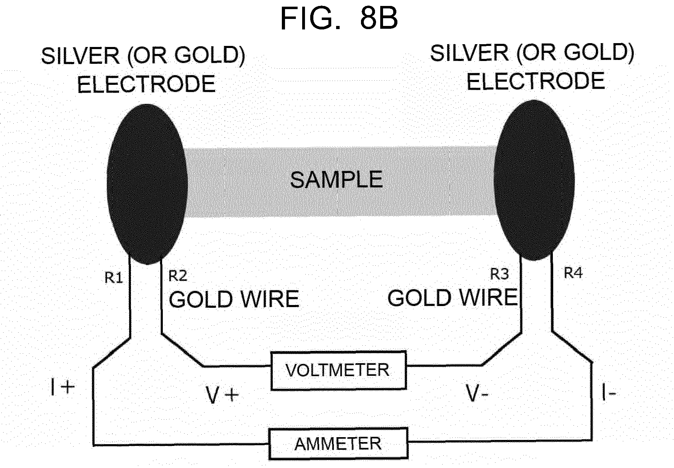

FIG. 8B is a drawing showing an arrangement of a sample and electrodes in a four-terminal method. In the four-terminal method, since the contact resistances R2 and R3 between the sample and a lead wire of a voltmeter are negligibly small compared to the internal resistance RV of the voltmeter, the influence of the contact resistances R2 and R3 does not have to be considered. When the voltage applied to the sample is read on the voltmeter, the contact resistances R1 and R4 between the sample and a lead wire of an ammeter do not affect the voltage applied to the sample, and thus these contact resistances can also be ignored (including the resistance of the lead wire itself).

The contact resistance depends on the junction to the sample, and is often on the order of, for example, approximately 0 to 100 Ω.

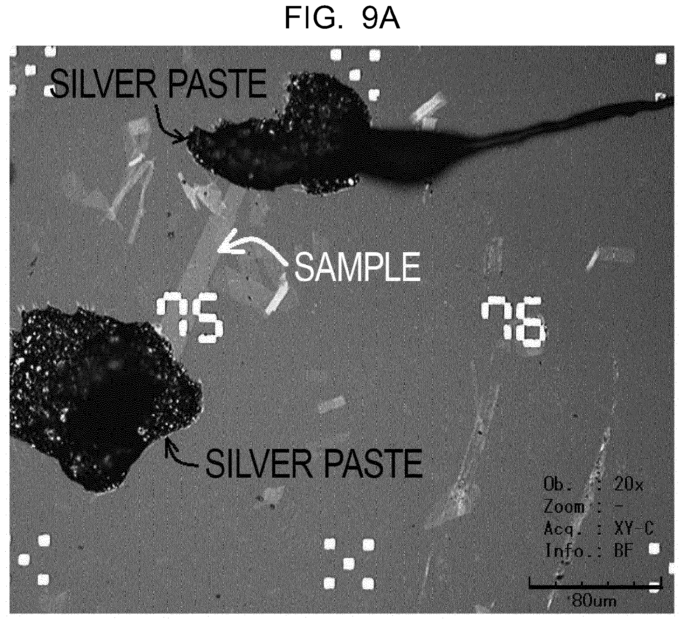

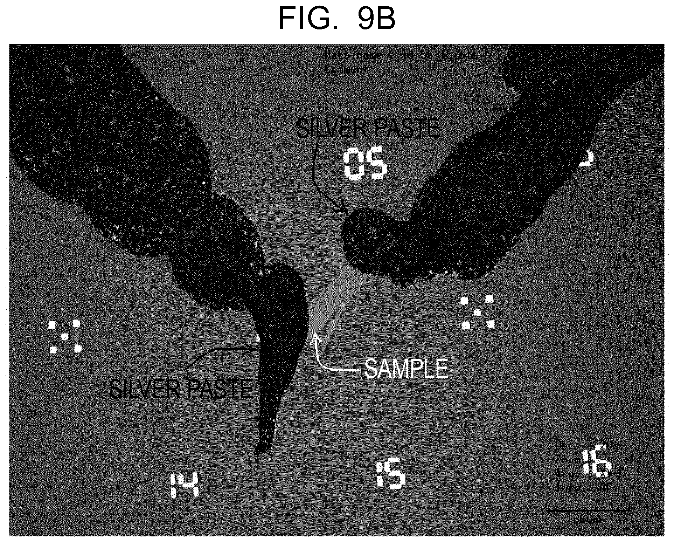

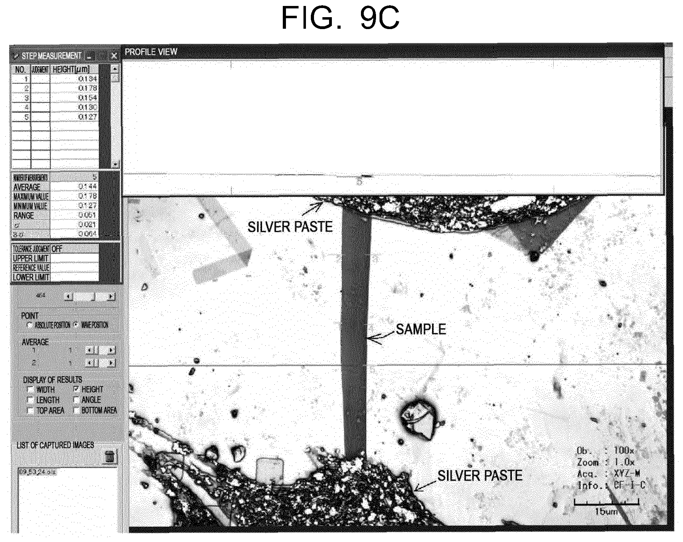

In order to subject the three types of TED single crystalline thin films having different film thicknesses, which were drop-cast on a silicon substrate insulatively coated with titanium oxide, to the measurement by the two-terminal method (FIG. 8A), two gold wires (0.01 mmΦ) as electrode probes were attached in the long axis direction using a silver paste (Dupont 4922N) at contact points (FIGS. 9A to 9C).

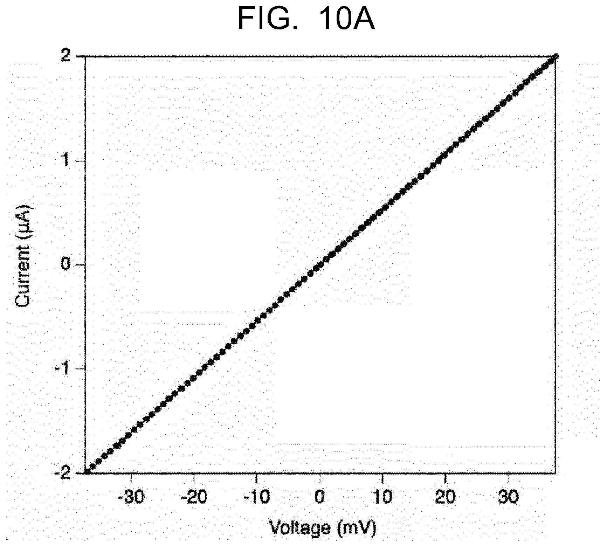

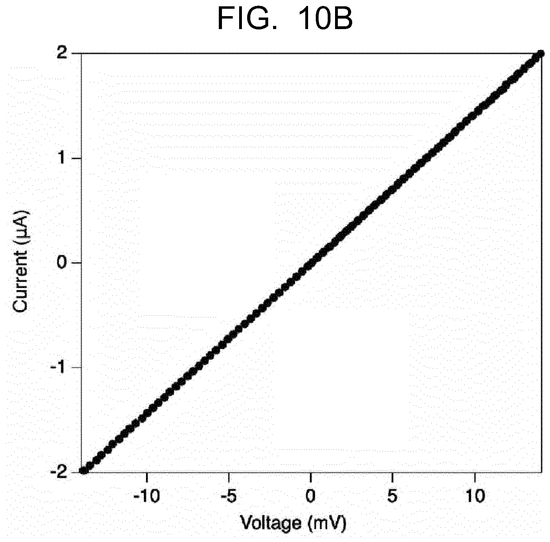

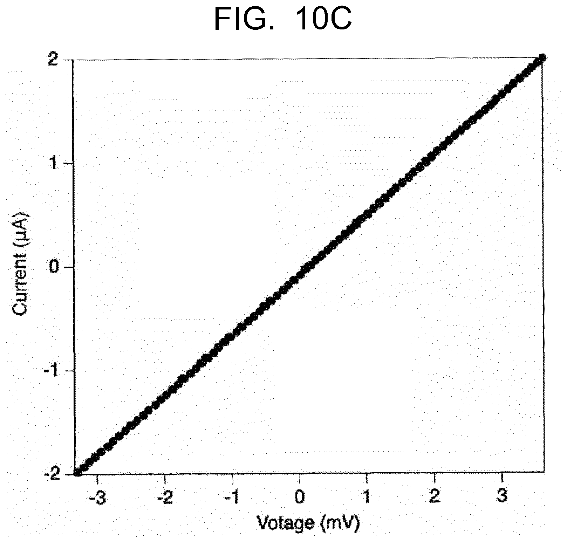

A change of the voltage value with respect to the applied current value was detected by Source Meter (KEITHLEY 2450) and Nanovoltmeter (KEITHLEY 2182A) at 300 K in a He cryostat. In all the cases, it was verified that the I-V characteristics were linear and the samples had ohmic contact with the electrodes (FIGS. 10A to 10C).

### <Measurement of resistivity (conductivity)>

The three types of TED thin films having different film thicknesses, which had been subjected to electrode attachment and electrical measurement by the method of evaluation of I-V characteristics described above, were subjected to measurement of conductivity at room temperature, and the change in conductivity with respect to the film thickness was plotted.

FIG. 11 is a graph showing a relationship of film thickness dependence of the room temperature conductivity, which shows examples of the present invention, where the horizontal axis represents the film thickness and the vertical axis represents the electrical conductivity [S/cm]. The electrical conductivity is 2700 [S/cm] at a film thickness of 1.6 nm, 520 [S/cm] at a film thickness of 8 nm, and 330 [S/cm] at a film thickness of 145 nm.

Since the measurement was performed by the two-terminal method, it is influenced by the contact resistance between the sample and the electrode, but a tendency was observed that the smaller the film thickness was, the higher the conductivity was.

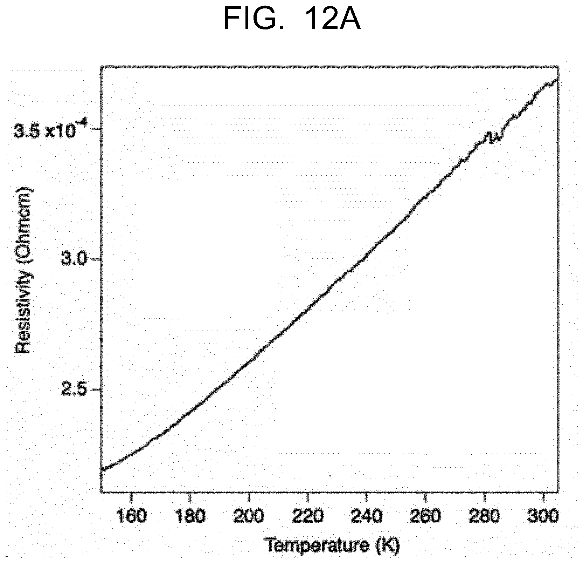

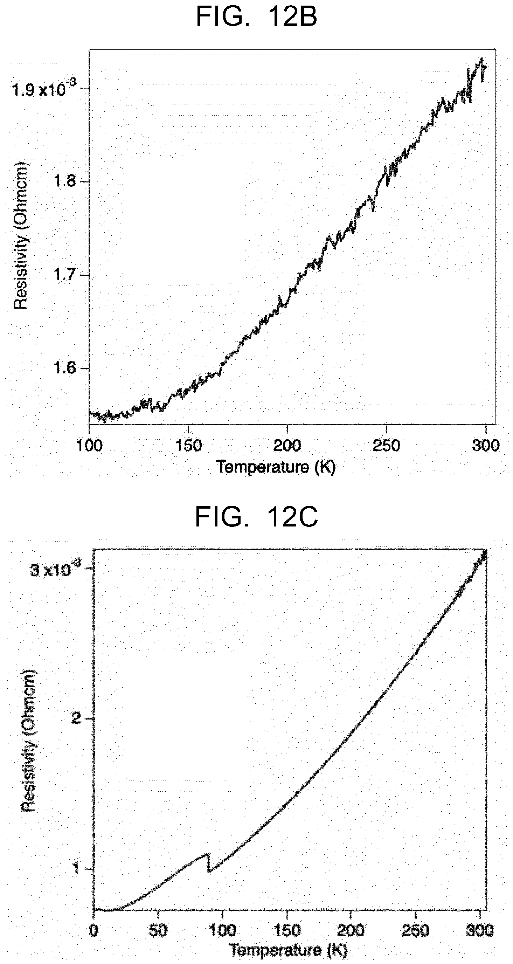

FIG. 12A shows the temperature dependence of resistivity of a TED monomolecular layer film having a film thickness of about 1.6 nm and having a terminal of a silver electrode attached to both the two ends, which shows an example of the present invention. FIG. 12B shows the temperature dependence of resistivity of a TED thin film having a film thickness of about 8 nm and having a terminal of a silver electrode attached to both the two ends. FIG. 12C shows the temperature dependence of resistivity of a TED thin film having a film thickness of about 145 nm and having a terminal of a silver electrode attached to both the two ends.

FIGS. 12A to 12C show the temperature dependence of resistivity of the three types of TED thin films having different film thicknesses that were subjected to electrode attachment and electrical measurement by the method of evaluation of I-V characteristics described above. Here, the vertical axis represents the resistivity (Ωcm), and the horizontal axis represents the temperature (K). All the thin films showed a metal conduction behavior in which the resistivity monotonously decreases with respect to the temperature in the measured temperature range.

### <Measurement using four-terminal method>

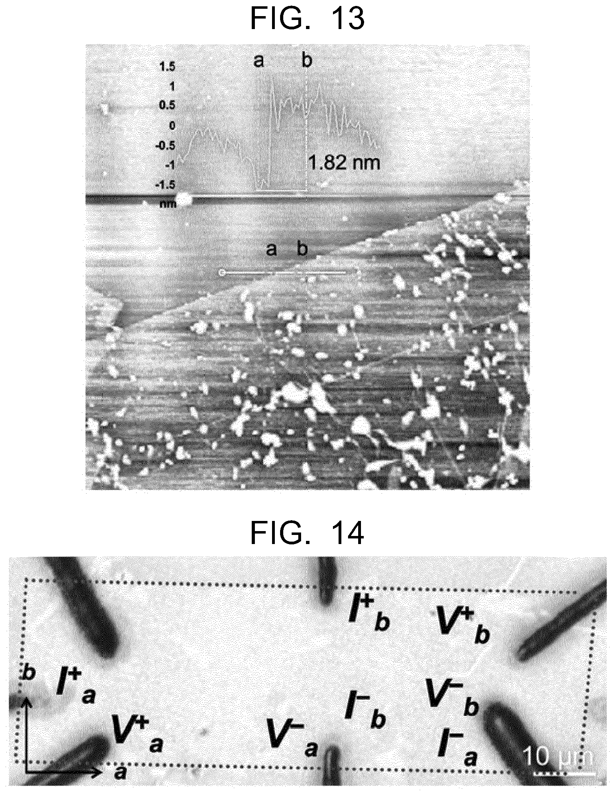

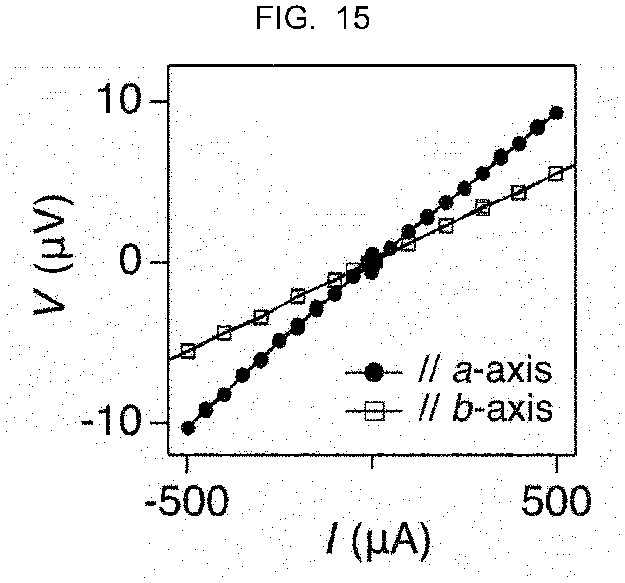

As a measurement specimen, a single crystalline TED monolayer film was prepared. The film had a size in the a-axis direction of 103 µm, a size in the b-axis direction of 32 µm, and a film thickness measured using an atomic force microscope (AFM) of 1.82 nm (see FIG. 13). As shown in FIG. 14, current terminals (Iₐ⁺, Iₐ⁻, I_{b}⁺, and I_{b}⁻) and voltage terminals (Vₐ⁺, Vₐ⁻, V_{b}⁺, and V_{b}⁻) were disposed on the measurement specimen in each of the a-axis direction and the b-axis direction. The current terminals and the voltage terminals were gold electrodes, and were formed by an inkjet printer using a water-soluble ink in which gold nanoparticles were dispersed. The contact resistance between the electrodes at this time was 133 Ω to 7.7 kΩ (see Table 2), and as shown in FIG. 15, the voltage showed an ohmic behavior for a current of ±500 mA.

**[Table 2]**

| | // a -axis (Ω) | // *b* -axis (Ω) |
|---|---|---|
| I⁺-I⁻ | 7.7 k | 316 |
| V⁺-V⁻ | 259 | 133 |

Since the single crystalline TED monolayer film has anisotropy, a current of 500 mA was applied in both the a-axis direction and the b-axis direction to perform four-terminal measurement. The resistivity (ρ) at room temperature was 3.4 nΩcm (a-axis direction) and 29.3 nΩcm (b-axis direction), and the conductivity (σ) was 0.29 GS/cm (a-axis direction) and 34 MS/cm (b-axis direction). Since it is generally known that gold has a resistivity of 2.4 mΩcm (20°C) and a conductivity of about 0.42 MS/cm (20°C), the single crystalline TED monolayer film has a conductivity nearly 100 to 1000 times higher than that of gold at room temperature. In addition, the conductivity of the single crystalline TED monolayer film is highest at normal temperature and normal pressure among those of known conductive substances. Moreover, as shown in FIG. 16, the temperature dependence of conductivity showed a behavior similar to that of metals in both the a-axis direction and the b-axis direction. It is to be noted that specimens other than the specimen used in this measurement also showed similar electrical characteristics to those shown in FIG. 16 in the case where they were single crystalline TED monolayer films of the same size, the electrodes were attached well, and the contact resistance was equivalent to that in Table 2.

### [II. Examples based on contents of P2]

### [Single crystal of compound A]

### 1. Synthesis of TED

A compound TED represented by Formula (IV) described above was synthesized with reference to the description in paragraphs 0105 to 0121 of Patent Literature 2 (the compound corresponds to the compound A). The structure of the synthesized compound was identified using ¹H-NMR (Nuclear Magnetic Resonance) and a time-of-flight mass spectrometer (electrospray ionization).

### 2. Production of TED single crystals (specimens (i) and (ii))

### (1) Specimen (i)

The synthesized TED powder (1 mg) was pulverized in a mortar and dissolved in dimethyl sulfoxide (DMSO, 160 µL) as a high-boiling-point organic solvent, and hydroxylamine (a 50 wt% solution, 50 µL) as a basic additive was added thereto to prepare a solution. Then, the solution was placed in a closed vial container and left to stand at room temperature for several months. A precipitate was observed in the solution after being left to stand. The precipitate was collected by filtration, washed with water in an amount of about 10 times the weight of the collected material, and then naturally dried to obtain a brownish specimen (i).

### (2) Specimen (ii)

A specimen (ii) was produced in the same manner as in the specimen (i) except that the percentage of hydroxylamine (a 50 wt% solution) to dimethyl sulfoxide (DMSO, 160 µL) was changed to 1 wt%.

### 3. Evaluation of physical properties of TED single crystals

### (1) Optical microscope observation and X-ray diffraction analysis (XRD analysis)

The specimens (i) and (ii) were observed with an optical microscope. The synthesized TED powder was composed of nanometer-scale pieces, and the specimen (i) obtained by subjecting the TED powder to the above-mentioned treatment was grown into a needle-like crystal having a long axis on a micrometer scale (about 400 µm × 20 µm × 10 µm) (see FIG. 1). Meanwhile, the specimen (ii) had a similar appearance to that of the TED powder before the treatment (see FIG. 2).

Then, the specimens (i) and (ii) were subjected to X-ray diffraction analysis (XRD analysis). FIG. 17 shows X-ray diffraction patterns of the specimen (i) (lower part) and the specimen (ii) (upper part). As for the specimen (i), a sharp peak was observed around a 2θ of 27°. Meanwhile, as for the specimen (ii), a peak was observed around a region of 25° to 28°, but the pattern was broad as a whole.

From the above-mentioned optical microscope observation and XRD analysis, it was verified that the specimen (i) was a single crystal of TED, and the specimen (ii) was amorphous

### TED.

### (2) X-ray crystallography of TED single crystal

Crystal structure analysis of the specimen (i) (TED single crystal) was performed by X-ray crystallography. The crystal structure analysis was performed using 1922 reflections in high resolution diffraction images collected at 113 K using a Rigaku Saturn CCD system. All atoms except hydrogen atoms were determined anisotropically.

The results of structure analysis are shown in FIGS. 3A to 3D. It was verified that the TED single crystal has a periodic structure in which two molecules are contained in an asymmetric unit, and has a layered crystal structure including molecular sheets stacked in the a-axis and b-axis directions. In FIG. 3A, the dashed lines indicate the intermolecular distance of S ... S contacts and an intramolecular hydrogen bond. In FIGS. 3B and 3C, each gray shaded portion indicates a single molecular layer. As illustrated in FIG. 3D, as for the overlapping modes of the π orbitals, the π orbitals are vertically overlapped by π ... π stacking along the a-axis and laterally overlapped by an S ... S contact along the b-axis. Values of the TED crystal lattice parameters are as follows: P2₁/m, a = 3.7464(2) Å, b = 11.8946(6) Å, c = 20.2094(11) Å, β = 93.506(2) Å, V = 898.88(8) Å, R₁ = 5.38%. The numerical values in parentheses indicate the error of the last digit in the X-ray structural analysis.

### 4. Production of TED single crystals (specimens 1 to 20)

Specimens 1 to 20 were prepared in the same manner as in the specimen (i) described above except that the types and ratios of the high-boiling-point organic solvent and the basic additive contained in the solution were changed. The types and ratios of the solvents used for preparing the specimens 1 to 20 are shown in Table 3.

The numerical values in Table 3 indicate weight ratios of the solvents with respect to the weight of the TED powder taken as 1. The high-boiling-point organic solvents and the basic additives in Table 3 mean the following.
DMSO: dimethyl sulfoxide
DMF: dimethylformamide
NMP: N-methylpyrrolidone
NH₂OH: aqueous hydroxylamine solution
NH₃: aqueous ammonia solution
NH(Me)₂: aqueous dimethylamine solution

**[Table 3]**

| Specimen No. | | High-boiling-point organic solvent | | | Basic additive | | |
|---|---|---|---|---|---|---|---|
| | Temperature(°C) | DMSO | DMF | NMP | NH₂OH | NH₃ | NH(Me)₂ |
| 1 | 25 | 0 | 169 | 0 | 0 | 67 | 0 |
| 2 | 25 | 145 | 0 | 0 | 45 | 0 | 0 |
| 3 | 25 | 0 | 169 | 0 | 54 | 0 | 0 |
| 4 | 25 | 145 | 0 | 0 | 45 | 0 | 0 |
| 5 | 25 | 364 | 0 | 0 | 214 | 0 | 0 |
| 6 | 25 | 145 | 0 | 0 | 134 | 0 | 0 |
| 7 | 25 | 145 | 0 | 0 | 89 | 0 | 0 |
| 8 | 25 | 91 | 0 | 0 | 179 | 0 | 0 |
| 9 | 25 | 91 | 0 | 0 | 9 | 0 | 0 |
| 10 | 25 | 218 | 0 | 0 | 67 | 0 | 0 |
| 11 | 25 | 145 | 0 | 0 | 45 | 0 | 0 |
| 12 | 25 | 73 | 0 | 0 | 22 | 0 | 0 |
| 13 | 25 | 145 | 0 | 0 | 45 | 0 | 0 |
| 14 | 25 | 36 | 0 | 0 | 11 | 0 | 0 |
| 15 | 25 | 131 | 17 | 0 | 54 | 0 | 0 |
| 16 | 25 | 15 | 152 | 0 | 54 | 0 | 0 |
| 17 | 25 | 73 | 85 | 0 | 54 | 0 | 0 |
| 18 | 25 | 116 | 34 | 0 | 54 | 0 | 67 |
| 19 | 25 | 29 | 136 | 0 | 54 | 0 | 0 |
| 20 | 25 | 136 | 0 | 29 | 54 | 0 | 0 |

The specimens 1 to 20 shown in Table 3 were subjected to optical microscope observation and XRD analysis in the same manner as in the above-mentioned specimens (i) and (ii). As a result, it was verified that the specimens 1 to 20 were also TED single crystals.

### [TED single crystalline thin films]

### 1. Production of TED single crystalline thin films

Specimens S1 to S5 having different film thicknesses shown in Table 4 were produced by the method described below. An aqueous lithium hydroxide solution (0.5 µL) at a pH of 9 to 11 as an aqueous alkali metal hydroxide solution was added to a TED single crystal (the specimen (i) described above, approximately 400 × 20 × 10 µm in size) to prepare a mixed liquid. The mixed liquid was left to stand at room temperature for at least 1 week. After the mixed liquid was left to stand, a large excess (50 µL) of water was added thereto, and the mixed liquid was further left to stand for 10 minutes. After being left to stand for 10 minutes, the mixed liquid was drop-cast on a glass substrate. The mixed liquid was naturally dried, and then washed twice with water (50 µL) heated to 50°C. After washing, the mixed liquid was naturally dried for 20 minutes to obtain a desired nanoscale single crystalline thin film (specimens S1 to S5).

The film thickness was measured using an atomic force microscope (AFM) (Bruker model MultiMode 8) and a silicon cantilever (SCANASYST-AIR) or Nano Search Microscope (SFT-3500 manufactured by Shimadzu Corporation).

In addition, a correlation between the film thickness and the number of layers (number of molecular layers) of the TED single crystalline thin films, which was calculated based on the X-ray crystallography result of the TED single crystal, is shown in FIGS. 7D and 7E. With reference to FIGS. 7D and 7E, the number of layers of the single crystalline thin films as the specimens S1 to S5 was determined, and is shown in Table 4.

**[Table 4]**

| Thin film specimen No. | Film thickness [nm] | Number of layers | Electrical conductivity [S/cm] |
|---|---|---|---|
| S1 | 1.6 | 1 | 2700 |
| S2 | 4 | 2 | - |
| S3 | 5 | 3 | - |
| S4 | 8 | 5 | 520 |
| S5 | 145 | 80 | 330 |

### 2. Evaluation of electrical characteristics of TED single crystalline thin films

The evaluation of electrical characteristics of the TED single crystalline thin films described below was performed by using a two-terminal method capable of measuring even a small specimen because the evaluation specimens were small. The resistance value measured by the two-terminal method reflects the contribution of both the specimens themselves and the contact resistance arising from a junction between the specimens and the electrode, but the two-terminal method is a sufficiently reliable method for the purpose of estimating the approximate resistance value of the specimens and determining the temperature dependence of resistance.

### (1) Voltage-current characteristics (I-V characteristics)

I-V characteristics of the specimens S1 (1.6 nm), S4 (8 nm), and S5 (145 nm) were evaluated by a two-terminal method. More specifically, a change of the voltage value with respect to the applied current value was detected by Source Meter (KEITHLEY 2450) and Nanovoltmeter (KEITHLEY 2182A) at 300 K in a He cryostat. The results are shown in FIGS. 10A to 10C, respectively. It was verified that the I-V characteristics of all of the specimens S1, S4, and S5 were linear and the specimens had ohmic contact with the electrodes.

### (2) Electrical conductivity

The electrical conductivity at room temperature of the specimens S1 (1.6 nm), S4 (8 nm), and S5 (145 nm) was evaluated by a two-terminal method. The results are shown in Table 4. On the basis of the measurement results, a change in electrical conductivity with respect to the film thickness is shown in FIG. 11. That is, the graph in FIG. 11 shows film thickness dependence of the electrical conductivity of the TED single crystalline thin films at room temperature. As can be understood from FIG. 11, it was verified that the electrical conductivity tends to increase as the film thickness of the TED single crystalline thin film decreases.

As described above, the two-terminal method was used in the evaluation of the electrical conductivity, but it is presumed that the four-terminal method may yield higher values (for example, values higher by two orders of magnitude).

### (3) Temperature dependence of electrical resistivity

The temperature dependence of electrical resistivity of the specimens S1 (1.6 nm), S4 (8 nm), and S5 (145 nm) was evaluated by a two-terminal method. The results are shown in FIGS. 12A to 12C. All the specimens S1, S4, and S5 showed a metal conduction behavior in which the resistivity monotonously decreases in line with a temperature drop in the measured temperature range.

### 3. Evaluation of optical characteristics of TED single crystalline thin films

### (1) Visible light transmittance and reflectance

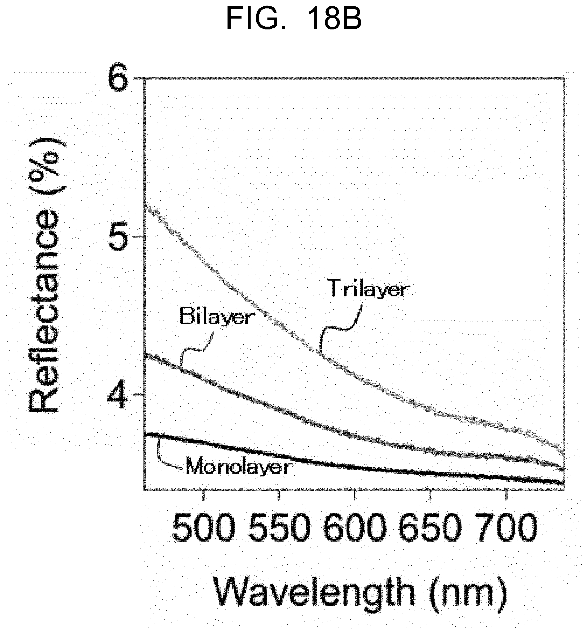

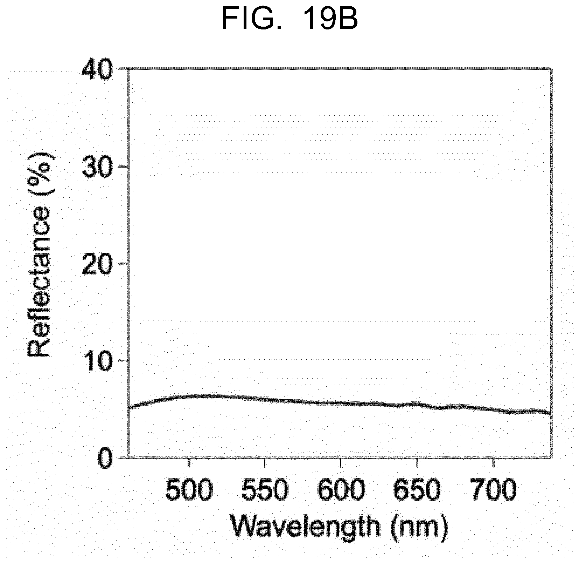

The visible light (wavelength: 450 to 750 nm) transmittance and reflectance were measured for the specimens S1 (monomolecular layer), S2 (bimolecular layer), and S3 (trimolecular layer). The results are shown in FIGS. 18A and 18B, respectively. FIG. 18A also shows the visible light transmittance of graphene (monolayer) for comparison. Furthermore, for comparison, the transmittance and reflectance of a single crystal of the compound A (needle-like single crystal, the specimen (i) described above), which is not a thin film, were measured. The results are shown in FIGS. 19A and 19B, respectively. The visible light transmittance and reflectance were measured at room temperature using a xenon light source (MAX-303, UV-VIS mirror module manufactured by Asahi Spectra Co., Ltd.) as a light source. In addition, since the specimen size was small, a 100x objective lens was used for the measurement of the specimens S1, S2, and S3, and a 20x objective lens was used for the measurement of the specimen (i). The reflectance is a value measured in a state where the thin film is placed on a glass substrate. The reflectance is measured at the position of the thin film specimen on the glass substrate, and corrected from the measured value of the glass substrate (and the refractive index).

As shown in FIG. 18A, the visible light transmittance of each of the specimens S1 (monomolecular layer) and S2 (bimolecular layer) was 98% or more, which was higher than the transmittance of the monolayer graphene. The visible light transmittance of the specimen S3 (trimolecular layer) was also high and 96% or more. As shown in FIG. 18B, the visible light reflectance of each of the specimens S1 to S3 was 6% or less. This is because, in the nanoscale single crystalline thin films (specimens S1 to S5), the plasma edge exists outside the visible light region unlike in general metals.

Graphene is a covalent sheet (film), and has a large absorption band in the visible light region due to its band structure even if it has a monolayer structure. In contrast, the specimens S1 to S3 are non-covalent sheets (films), do not have a large absorption band in the visible light region, and further, do not have a plasma edge in the visible light region, so that it is presumed that the specimens S1 to S3 realized transparency exceeding that of graphene.

Meanwhile, as shown in FIG. 19A, the visible light transmittance of the specimen (i) (needle-like single crystal) was as low as 50% or less. In addition, the specimen (i) had a large absorption band in the visible light region and was colored in reddish brown. From the comparison between FIG. 18A and FIG. 19A, it was verified that as for the crystal of the compound A, the transmittance was improved due to thinning of the crystal, and the large absorption band in the visible light region was not found and the crystal was colorless. As shown in FIG. 19B, the visible light reflectance of the specimen (i) was 10% or less.

### (2) Ultraviolet light transmittance and reflectance

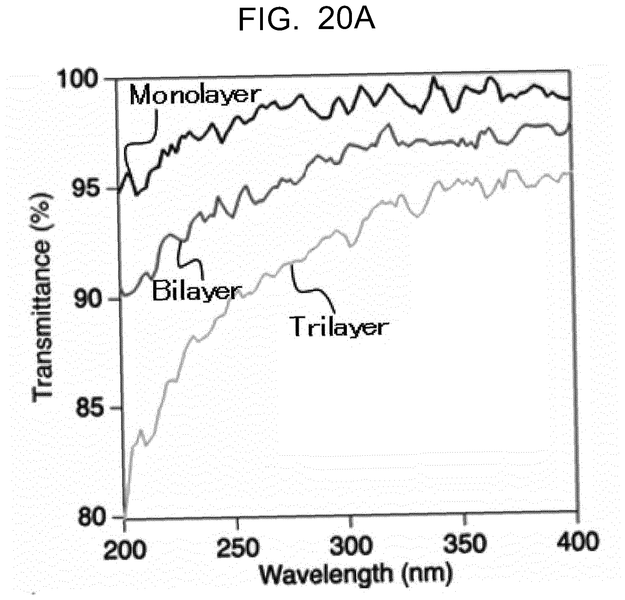

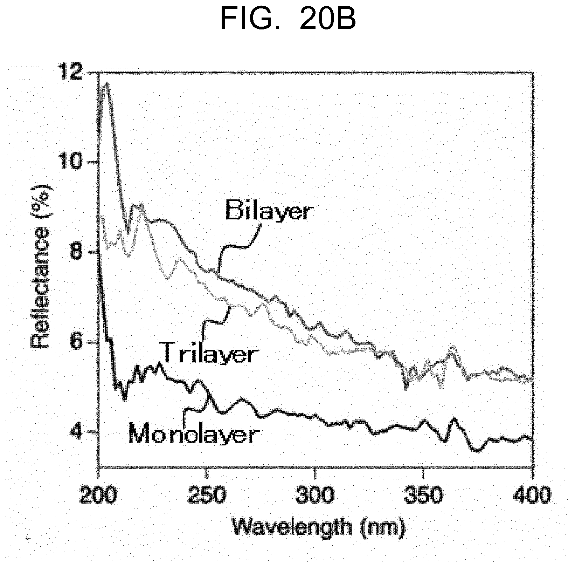

The ultraviolet light (wavelength: 200 to 400 nm) transmittance and reflectance were measured for the specimens S1 (monomolecular layer), S2 (bimolecular layer), and S3 (trimolecular layer). The results are shown in FIGS. 20A and 20B, respectively. The ultraviolet light transmittance and reflectance were measured using UV/VIS Microscopic Spectrophotometer (MSV-5700 manufactured by JASCO Corporation).

As shown in FIG. 20A, the ultraviolet light transmittance of each of the specimens S1 (monomolecular layer) and S2 (bimolecular layer) was 90% or more, and the ultraviolet light transmittance of the specimen S3 (trimolecular layer) was also high and 80%. As shown in FIG. 20B, the ultraviolet light reflectance of each of the specimens S1 to S3 was 12% or less. From these results, it was verified that the specimens S1 to S3 do not have a large absorption band in the deep ultraviolet light region (wavelength: 200 to 360 nm), that is, are transparent, and have a transmittance and a reflectance in this region comparable to those of glass.

### INDUSTRIAL APPLICABILITY

A TED thin film as a single-component organic molecular metallic (molecular metal) single crystalline thin film of the present invention, which is based on the contents of P1 or P3, is an ideal electrode material having both high conductivity and ultra-thin film properties, and is suitable for various electrode materials including materials of a transparent electrode.

The method for manufacturing a single-component organic molecular metallic (molecular metal) single crystalline thin film of the present invention, which is based on the contents of P1 or P3, can form a film using, for example, only drop casting, has a simple production process, can increase the yield of a product, and contributes to, for example, the promotion of industrial use of a printable electrode.

The single crystalline thin film of a synthetic metal (molecular metal) of the present invention, which is based on the contents of P2, has high electrical conductivity, high light transmissivity, and ultra-thin film properties. The single crystalline thin film of a synthetic metal of the present invention is suitable for various electrode materials including materials of a transparent electrode, for example.

## Claims

1. A single crystalline thin film of a molecular metal, the single crystalline thin film comprising any one compound represented by a general formula selected from the group shown below: 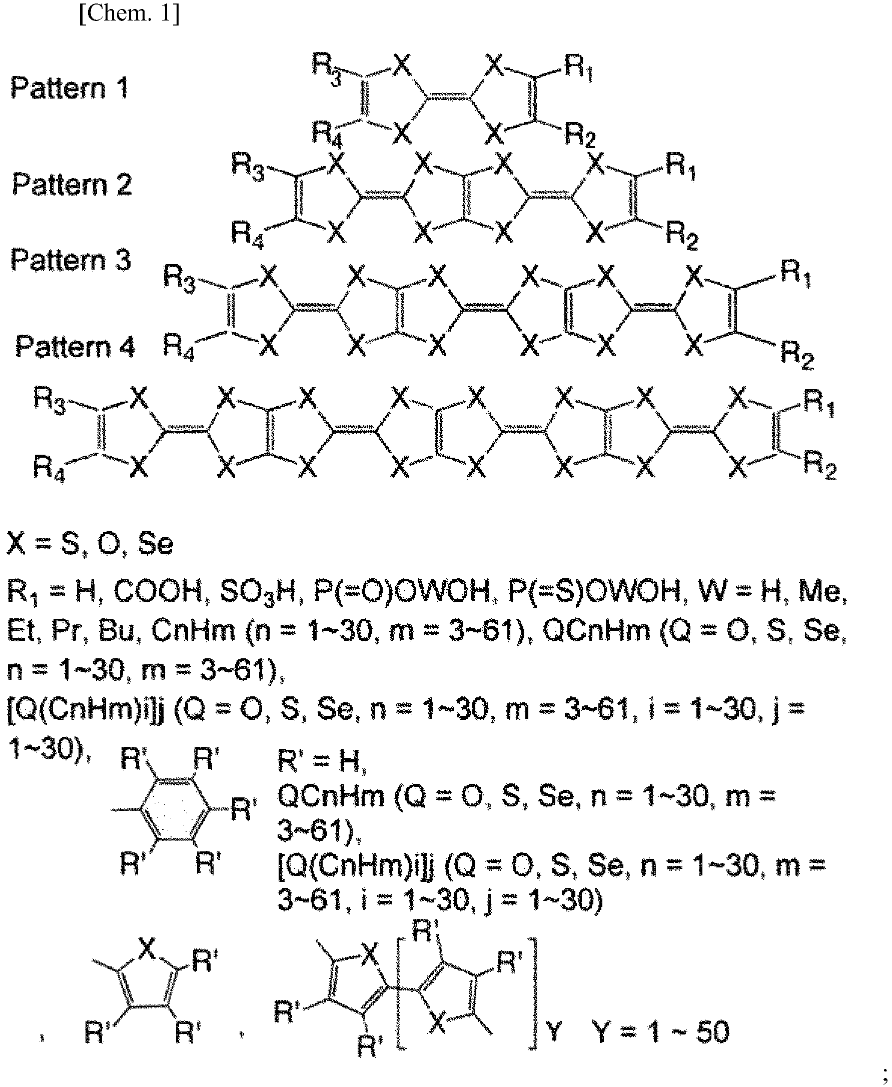 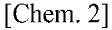 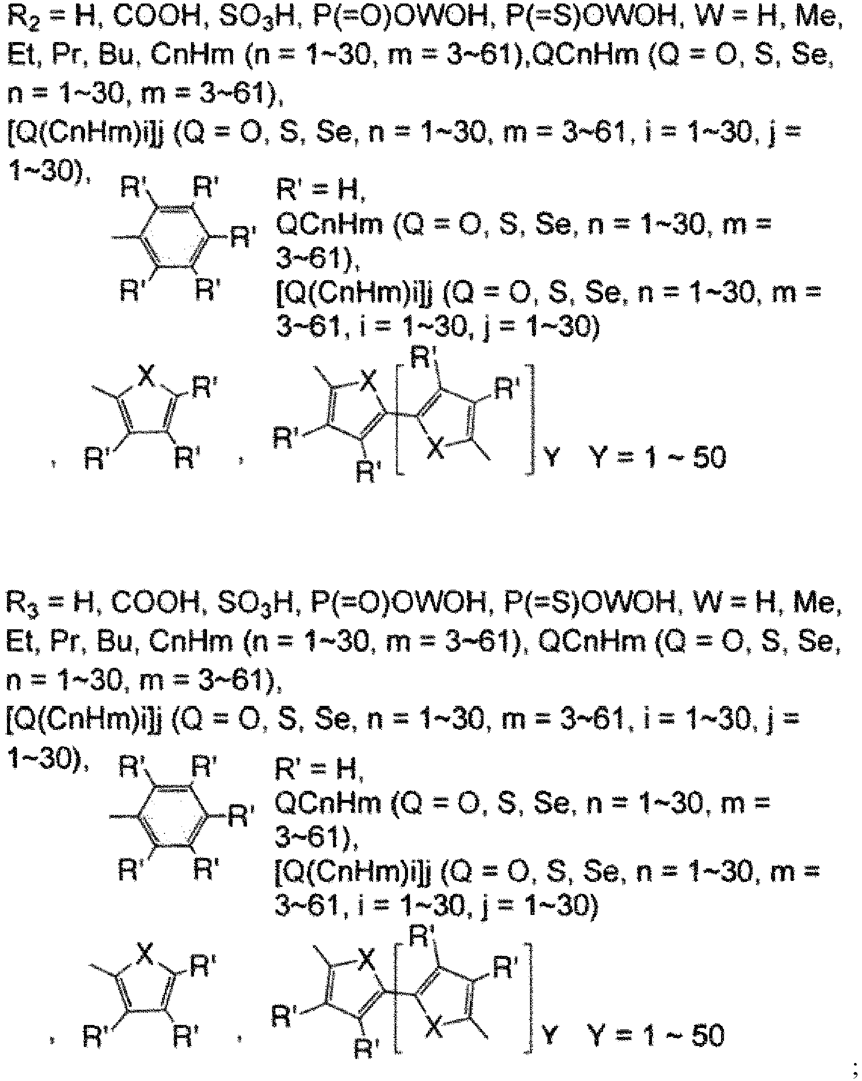 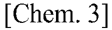 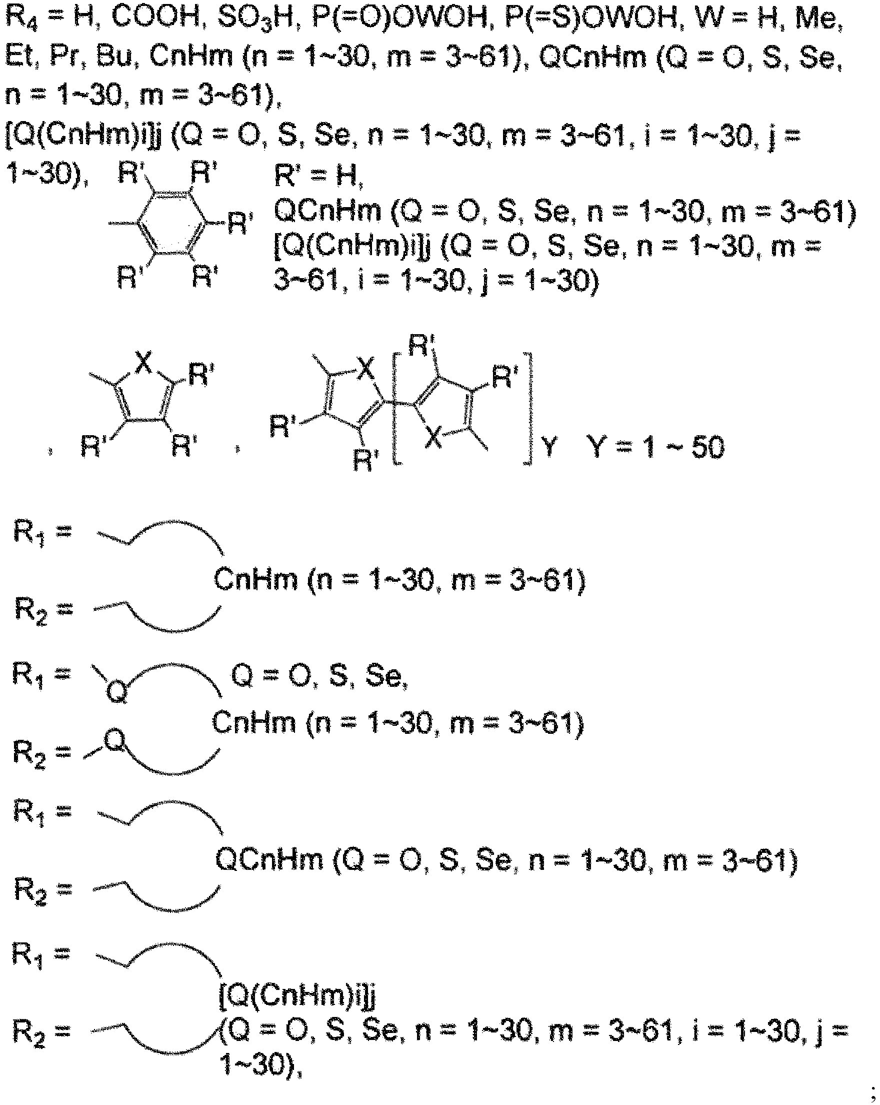 and 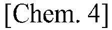 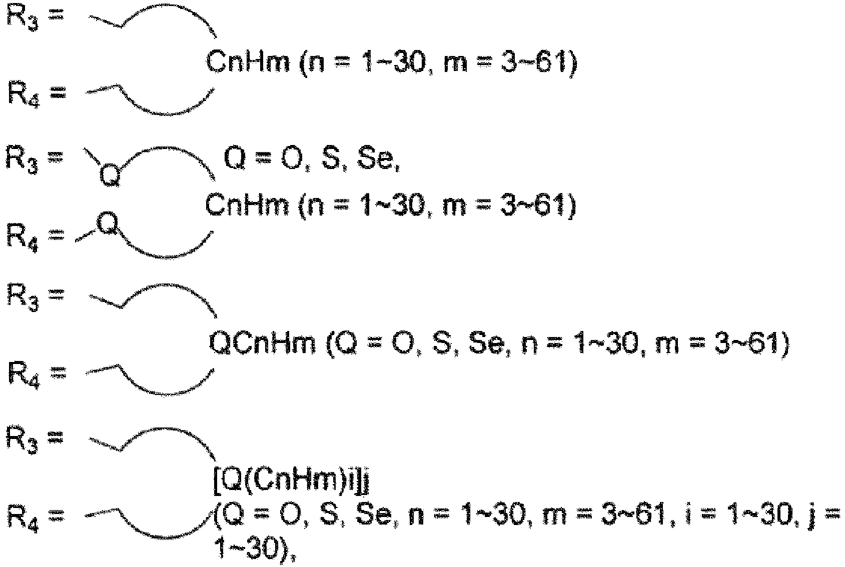 wherein R₁, R₂, R₃, R₄, and R' may be identical or different.

2. A single crystalline thin film of a molecular metal, the single crystalline thin film comprising a compound represented by a general formula shown below:

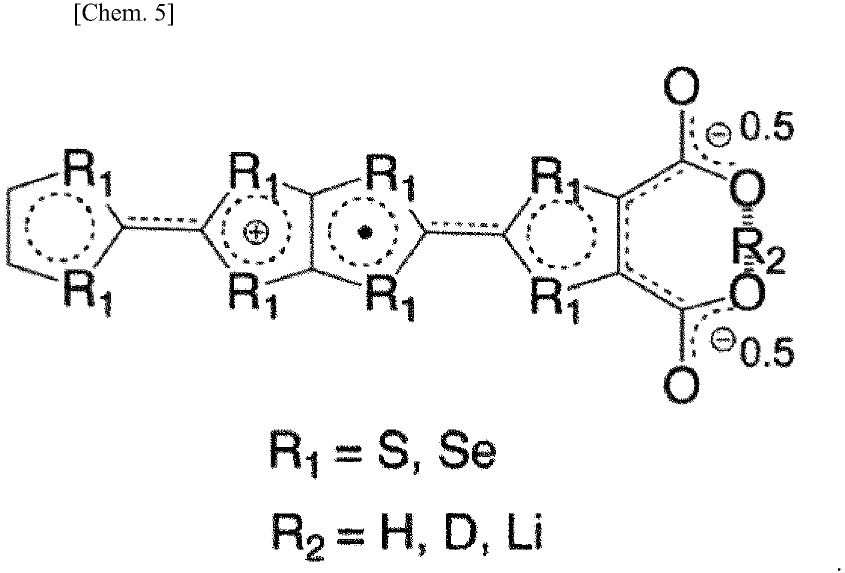

3. The single crystalline thin film according to claim 1 or 2, having an electrical conductivity of 1 × 10⁻³ to 1 × 10⁷ S/cm.

4. The single crystalline thin film according to any one of claims 1 to 3, having a film thickness of 1 nm or more and 1000 nm or less.

5. The single crystalline thin film according to any one of claims 1 to 4, having an electrical conductivity measured using a four-terminal method of 1 × 10 to 1 × 10¹² S/cm.

6. The single crystalline thin film according to any one of claims 1 to 5, wherein the molecular metal is a compound represented by Formula (I) shown below: 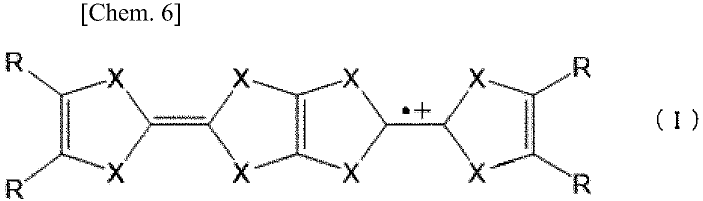
wherein X represents at least one atom selected from the group consisting of S, O, and Se, and a plurality of Xs may be identical or different, and
R represents a hydrogen atom or a monovalent substituent, and a plurality of Rs may be identical or different, provided that at least one of Rs is at least one group selected from the group consisting of groups represented by Formula (II) shown below, and Rs substituted with adjacent atoms may be linked to each other to form a ring: 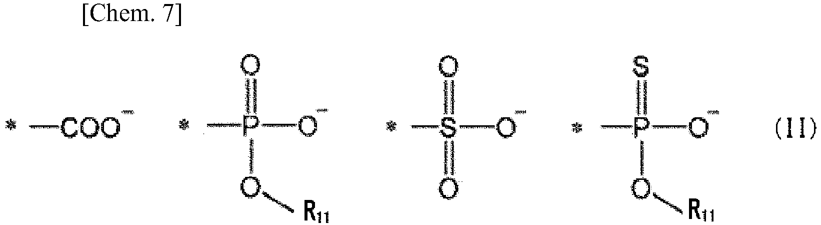
wherein * represents a bonding position, and R₁₁ represents a hydrogen atom or an alkyl group having 1 to 4 carbon atoms.

7. The single crystalline thin film according to any one of claims 1 to 6, having a transmittance of light with a wavelength of 360 to 830 nm of 80% or more.

8. The single crystalline thin film according to any one of claims 1 to 7, having a transmittance of light with a wavelength of 200 to 360 nm of 60% or more.

9. The single crystalline thin film according to any one of claims 1 to 8, having a film thickness of 1 to 20 nm.

10. The single crystalline thin film according to any one of claims 1 to 9, comprising a mono- to deca-molecular layer of a compound represented by Formula (I).

11. The single crystalline thin film according to any one of claims 1 to 10, wherein the molecular metal is a compound represented by Formula (III) shown below: 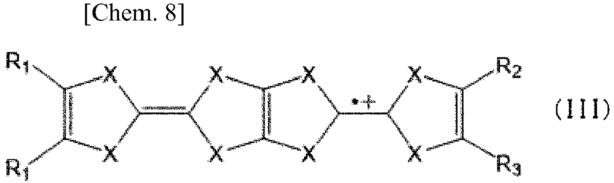
wherein X represents an atom of S or Se, and a plurality of Xs may be identical or different,
R₁ represents a hydrogen atom, a hydrocarbon group, a (poly)alkyleneoxy group, or a (poly)alkylenethio group, and two Ris may be identical or different, and
either of R₂ and R₃ is a group represented by Formula (II), and the other is a Bronsted acid group corresponding to the group represented by Formula (II), or a salt thereof.

12. The single crystalline thin film according to any one of claims 1 to 11, wherein the molecular metal is a compound represented by Formula (IV) shown below:

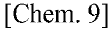

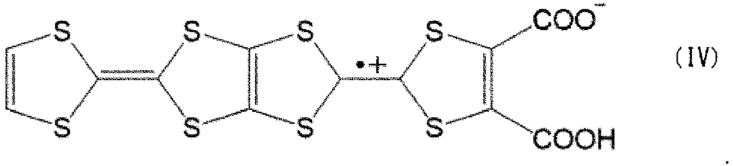

13. The single crystalline thin film according to any one of claims 1 to 12, having an electrical conductivity according to evaluation by a two-terminal method of 1 × 10⁻³ to 1 × 10⁷ S/cm.

14. A component comprising the single crystalline thin film according to any one of claims 1 to 13,
the component being an optical component or an electronic component.

15. The component according to claim 14, which is a transparent electrode or an optical fiber.

16. A method for manufacturing the single crystalline thin film according to any one of claims 1 to 13, the method comprising:
preparing a mixed liquid containing a single crystal of the molecular metal and an aqueous alkali metal hydroxide solution;
leaving the mixed liquid to stand for a predetermined time;
bringing a base material into contact with the mixed liquid after being left to stand to form the single crystalline thin film on the base material; and
washing the single crystalline thin film formed on the base material.

17. The manufacturing method according to claim 16, wherein the predetermined time for leaving the mixed liquid to stand is 168 hours or more.

18. The manufacturing method according to claim 16 or 17, wherein in the mixed liquid, a weight ratio of the aqueous alkali metal hydroxide solution to the single crystal of the molecular metal is 2 to 1 × 10⁶.

19. The manufacturing method according to any one of claims 16 to 18, wherein the aqueous alkali metal hydroxide solution has a pH of 7.5 to 11.

20. The manufacturing method according to any one of claims 16 to 19, wherein by leaving of the mixed liquid to stand, in the mixed liquid, a synthetic metal piece as a monomolecular layer, crystallinity of the monomolecular layer in a two-dimensional direction being maintained, is exfoliated from the single crystal of the molecular metal.
